# EUROPÄISCHE PATENTSCHRIFT

(11) **EP 2 686 298 B1**
(45) Veröffentlichungstag und Bekanntmachung des Hinweises auf die Patenterteilung: **09.05.2018**
(21) Anmeldenummer: 12724856.5
(22) Anmeldetag: 12.03.2012
(51) Int. Cl.: C07C 305/24, C07C 307/02, C07D 311/12, A61P 35/00, A61K 31/37, A61K 31/655, A61K 31/255

(54) **SUBSTITUIERTE DIPHENYLDERIVATE**
SUBSTITUTED DIPHENYL DERIVATIVES
DÉRIVÉS DE DIPHENYLE SUBSTITUÉS

(30) Priorität: 16.03.2011 DE 102011014087
(43) Veröffentlichungstag der Anmeldung: 22.01.2014
(73) Patentinhaber: Creative Therapeutics GmbH, 42113 Wuppertal (DE)
(72) Erfinder: FÖRSTER, Heinz, 56337 Kadenbach (DE)
(74) Vertreter: Von Rohr Patentanwälte Partnerschaft mbB
(86) Internationale Anmeldenummer: PCT/DE2012/000258
(87) Internationale Veröffentlichungsnummer: WO 2012/122969

(56) Entgegenhaltungen:
- WO-A1-2009/004060
- US-A- 2 648 660

## Beschreibung

Die vorliegende Erfindung betrifft substituierte Diphenylderivate, die pro Molekül mindestens (i) eine Dialkyltriazenyl-, (ii) mindestens eine Sulfooxy- und/oder mindestens eine Sulfamoyloxygruppe enthalten, deren Salze, Solvate und die Solvate dieser Salze. Die Erfindung betrifft ferner Verfahren zur Herstellung dieser Verbindungen und ihre Verwendung als Arzneimittel.

Im Rahmen der vorliegenden Erfindung sind Diphenylderivate als durch eine einfache Bindung oder durch ein Brückenglied verbundene Phenylringe zu verstehen.

Triazen-substituierte Diphenylderivate sind bekannt. So offenbaren die DE 17 93 115 A1, die DE 21 47 781 A1 und die WO 2004/106358 A1 unter anderem Diphenylderivate, die mit Triazengruppen und z. B. Sulfonsäure- oder Oxycarbonsäuregruppen substituiert sind.

Triazenderivate wurden in den vergangenen Jahrzehnten ausführlich auf ihre zytostatische Wirksamkeit untersucht. Diese herkömmlichen Triazenzytostatika gehören zu den Alkylantien und sind aufgrund ihrer starken Nebenwirkungen und Toxizität nie zu einer breiteren Anwendung in der Klinik gekommen. Eine Ausnahme stellt das Dacarbazin (DTIC) dar, das ein Prodrug von Monomethyltriazeno-imidazol-carboxamid (MTIC) ist und hauptsächlich zur Bekämpfung von Morbus Hodgkin und Weichteilsarkomen eingesetzt wird (Cancer Treatment Reports 60, 205-211 (1976)). Wegen der Lichtempfindlichkeit von Dacarbazin und insbesondere seiner Nebenwirkungen, bei denen Leuko- und Thrombopenie besonders wesentlich sind, wurde eine großen Anzahl von Arylalkyltriazenen mit dem Ziel untersucht, potentere und besser verträglichere Triazene zu synthetisieren (Cancer Treatment Reports 60: 125-134 (1976); J. Med. Chem. 23, 1052-1024 (1980)). Trotz dieser Anstrengungen sind bis heute Dacarbazin und Temozolomid die einzigen Triazene zur Behandlung von Glioblastomen in der klinischen Anwendung geblieben, obwohl sie mit erheblichen Nebenwirkungen wie Knochenmarksdepression, Neuro- und Lebertoxizität, Erbrechen, Haarausfall und Exanthemen behaftet sind.

Ein erster Versuch zur Überwindung der Verträglichkeitsproblematik von ausgewählten Triazenen hin zur selektiven Anwendung beim Mammakarzinom wird in DE 17 93 115 A1 und DE 21 47 781 A1 beschrieben.

Ein zweiter Versuch zur Überwindung schwerer Nebenwirkungen von ausgewählten Triazenen wird in der WO 2004/106358 A1 beschrieben. Durch Einführung von Oxycarbonsäuregruppen sollen, einige schwere Nebenwirkungen der bekannten Triazene gemildert werden. Wegen unerwünschten Nebenwirkungen auf die Niere sind sie allerdings für eine Langzeitanwendung nicht geeignet.

Die WO 2009/004060 A1 betrifft Triazinverbindungen sowie Verfahren zu ihrer Herstellung. Weiterhin werden die Triazinverbindungen umfassende pharmazeutische Zusammensetzungen sowie ihre Verwendung zur Behandlung von Krebserkrankungen beim Menschen offenbart. Die Triazinverbindungen sollen gegenüber anderen Triazinverbindungen eine verbesserte Wirksamkeit bei gleichzeitig verringerter Toxizität aufweisen.

Die Aufgabe der vorliegenden Erfindung besteht darin, für eine Langzeittherapie geeignete onkozide Wirkstoffe praktisch ohne toxische Nebenwirkungen und mit gleichzeitig verbesserter Wirksamkeit bereitzustellen.

Es wurden neue Diphenylderivate, die pro Molekül (i) mindestens eine Dialkyltriazenyl- und (ii) mindestens eine Sulfooxy- und/oder mindestens eine Sulfamoyloxygruppe enthalten, und deren Salze, Solvate und Solvate dieser Salze gefunden.

Die erfindungsgemäßen neuen Diphenylderivate haben überraschenderweise eine stark verbesserte antitumorale Wirkung bei Tumoren wichtiger Zielorgane (z. B. Colon, Lunge, Leber, Pankreas, Niere) im Besonderen als selektive Onkozide gegen Tumoren der Haut, Brust, Prostata und Hoden sowie der von Sexualhormonen abhängigen Zielorgane eine stark verbesserte Wirksamkeit im Vergleich zu ähnlichen bekannten Wirkstoffen (WO 2004/106358 A1,

DE 17 93 115 A1 und DE 21 47 781 A1). Im Vergleich zu ähnlichen bekannten Wirkstoffen sind sie praktisch ohne toxische Nebenwirkungen und damit für eine Langzeittherapie geeignet.

Aufgrund ihrer besseren Verträglichkeit bei hoher Wirkungsintensität können sie sowohl als Monotherapie als auch in Kombination mit anderen Kanzerostatika, tumorspezifischen Antikörpern, bzw. Antikörper, die über Linker mit den erfindungsgemäßen Verbindungen gekoppelt sind medizinisch sinnvoll eingesetzt werden.

Intrazellulär setzen sie ein proliferationshemmendes/onkozides Wirkprinzip frei, das den unkontrollierten Replikationsstoffwechsel der Tumorzellen stoppen und sogar zu einer Regression des Tumors führen kann.

Die neuen Diphenyl-Derivate können als Wirkstoffe in Arzneimittel für Mensch und Tier eingesetzt werden.

Diphenyl-Triazene mit Schwefelsäure- und Schwefelsäureamidgruppen im Molekül sind nicht beschrieben und es gibt keinen Hinweis auf eine besondere Wirksamkeit.

Im Rahmen der vorliegenden Erfindung werden folglich Diphenylderivate der Formel 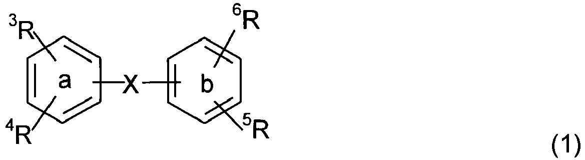 worin
- X: CO oder
- R³, R⁶: unabhängig voneinander Wasserstoff, Hydroxyl-, C₁-C₄-Alkyl, C₁₋C₄-Alkoxy, C₁-C₄-Alkyl-S-, C₁-C₄-Alkyl-SO-, C₁-C₄-Alkyl-SO₂-, Halogen, Nitro, Cyano, oder eine -OSO₂Y-Gruppe,
- R⁴, R⁵: unabhängig voneinander eine -N=N-N(R²)₂- oder eine -OSO₂Y-Gruppe,
- R⁷: Wasserstoff, Methyl, Ethyl oder einen durch die Reste R⁹, R¹⁰ substituierten Phenylrest, der direkt an das R⁷-tragende C-Atom geknüpft ist,
- R⁸: Wasserstoff, Ethyl, CN, NO₂, -CH₂CH₂-Halogen oder CH₂CH₂OH,
- R⁹, R¹⁰: unabhängig voneinander Wasserstoff, Hydroxyl-, C₁-C₄-Alkyl, C₁-C₄-Alkoxy, Halogen, Nitro, Cyano, eine -N=N-N(R²)₂- oder eine -OSO₂Y-Gruppe,
- Y: OH oder N(R¹)₂,
- R¹: Wasserstoff, Methyl oder Ethyl und
- R²: Methyl oder Ethyl
bedeuten,
mit der Maßgabe, dass pro Gesamtmolekül (1) ein bis zwei -N=N-N(R²)₂- und ein bis zwei -OSO₂Y-Gruppen an beliebigen Ring-Kohlenstoffatomen von aromatischen Ringen stehen, sowie deren Salze, freie Säuren, Solvate und die Solvate dieser Salze und die Solvate dieser freien Säuren eingesetzt.

Es wurde auch ein Verfahren zur Herstellung der neue Diphenylderivate, die pro Molekül (i) mindestens eine Dialkyltriazenyl- und (ii) mindestens eine Sulfooxy- und/oder mindestens eine Sulfamoyloxygruppe enthalten, und deren Salze, Solvate und Solvate dieser Salze gefunden, das dadurch gekennzeichnet ist, dass Aminophenylderivate der Formel 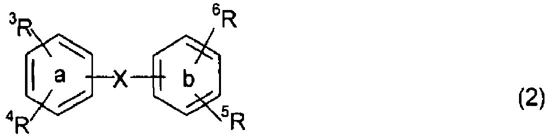 worin
- X: CO oder
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -NH₂- oder eine -OSO₂Y-Gruppe,
- R⁷: Wasserstoff, Methyl, Ethyl oder einen durch die Reste R⁹, R¹⁰ substituierten Phenylrest, der direkt an das R⁷-tragende C-Atom geknüpft ist,
- R⁸: Wasserstoff, Ethyl, CN, NO₂, -CH₂CH₂-Halogen (F, Cl, Br, J) oder CH₂CH₂OH,
- R⁹, R¹⁰: unabhängig voneinander Wasserstoff, Hydroxyl, C₁-C₄-Alkyl, vorzugsweise Methyl oder Ethyl, C₁-C₄-Alkoxy, vorzugsweise Methoxy oder Ethoxy, Halogen (F, Cl, Br, J), Nitro, Cyano, eine -NH₂- oder eine -OSO₂Y-Gruppe,
- Y: OH oder N(R¹)₂ und
- R¹: Wasserstoff, Methyl oder Ethyl bedeuten
mit der Maßgabe, dass pro Gesamtmolekül (2) ein bis zwei NH₂- und ein bis zwei -OSO₂Y-Gruppen an beliebigen Ring-Kohlenstoffatomen der aromatischen Ringen stehen
in Gegenwart starker Säure bei niedriger Temperatur, vorzugsweise 0 bis 8, besonders bevorzugt 0 bis 5 und insbesondere 0 bis 2°C in wässrig-saurer Lösung mit einem Diazotierungsmittel zu Diazoniumsalzen der Formel 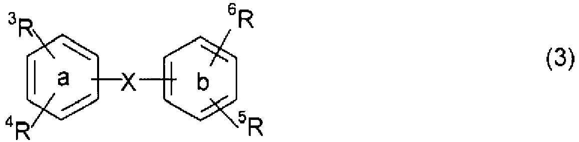 worin
- X: CO oder -CR⁷=CR⁸-

- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -N₂⁺An⁻- oder eine -OSO₂Y-Gruppe,
- R⁷: Wasserstoff, Methyl, Ethyl oder einen durch die Reste R⁹, R¹⁰ substituierten Phenylrest, der direkt an das R⁷-tragende C-Atom geknüpft ist,
- R⁸: Wasserstoff, Ethyl, CN, NO₂, -CH₂CH₂-Halogen (F, Cl, Br, J) oder CH₂CH₂OH,
- R⁹, R¹⁰: unabhängig voneinander Wasserstoff, Hydroxyl, C₁-C₄-Alkyl, C₁-C₄-Alkoxy, worin Alkyl vorzugsweise für Methyl oder Ethyl steht, Halogen (F, Cl, Br, J), Nitro, Cyano, eine -N₂⁺An⁻- oder eine -OSO₂Y-Gruppe,
- An⁻: ein Halogen (Cl⁻, Br⁻)- oder ½ Sulfat (SO₄²⁻)-Anion,
- Y: OH oder N(¹R)₂ und
- R¹: Wasserstoff, Methyl oder Ethyl bedeuten
mit der Maßgabe, dass pro Gesamtmolekül (3) ein bis zwei Diazonium- und ein bis zwei -OSO₂Y-Gruppen an beliebigen Ring-Kohlenstoffatomen von aromatischen Ringen stehen,
diazotiert,
und dann die erhaltenen Diazoniumsalze (3) mit Dialkylaminen der Formel

HN(R²)₂ (4)

worin R² für Methyl oder Ethyl steht,
in Gegenwart von Säurebindemittel umgesetzt werden,
gegebenenfalls gefolgt durch Herstellung der Salze aus den erhaltenen Verbindungen und gegebenenfalls gefolgt durch Freisetzung der Hydroxyl- und/oder Säuregruppen aus diesen Salzen.

Als Verdünnungsmittel kommen bei der Diazotierungsreaktion alle unter Reaktionsbedingungen inerten polaren Lösungsmittel in Frage, z. B. Alkohole wie Methanol, Ethanol, ferner Dimethylformamid und Dimethylsulfoxid und insbesondere Wasser oder Gemische dieser Lösungsmittel.

Als starke Säure bei der Diazotierungsreaktion können Halogenwasserstoffsäuren, vorzugsweise Salzsäure oder Schwefelsäure, Verwendung finden.

Als Diazotierungsmittel werden vorzugsweise Kaliumnitrit und Natriumnitrit genannt.

Als Säureakzeptoren bei der Kupplungsreaktion können alle üblichen Säurebindemittel verwendet werden. Hierzu gehören vorzugsweise Natrium- und Kaliumcarbonat.

Salze im Rahmen der Erfindung sind vorzugsweise physiologisch unbedenkliche Salze. Sie umfassen Salze üblicher Basen, wie beispielsweise Alkalimetallsalze (z. B. Natrium- und Kaliumsalze), Erdalkalimetallsalze (z. B. Calcium- und Magnesiumsalze) und Ammoniumsalze, abgeleitet von Ammoniak oder organischen C₁-C₁₆-Aminen, wie beispielsweise Ethylamin, Diethylamin, Triethylamin, Ethyldiisopropylamin, Monoethanolamin, Diethanolamin, Tris-hydroxyethylamin, Dicyclohexylamin, Dimethylaminoethanol, Dibenzylamin, N-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Alternativ kann man zur Herstellung der erfindungsgemäßen Diphenylderivate auch zuerst die Triazenyl- und erst dann Sulfooxy- und/oder Sulfamoyloxygruppen einführen. Zu diesem Zweck kann man Triazenylphenole der Formel 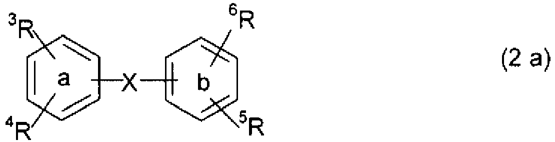 worin
- X: CO oder -CR⁷=CR⁸-
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine OH-Gruppe,
- R⁴, R⁵: unabhängig voneinander eine -N=N-N(R²)₂- oder eine OH-Gruppe,
- R⁷: Wasserstoff, Methyl, Ethyl oder einen durch die Reste R⁹, R¹⁰ substituierten Phenylrest, der direkt an das R⁷-tragende C-Atom geknüpft ist,
- R⁸: Wasserstoff, Ethyl, CN, NO₂, -CH₂CH₂-Halogen (F, Cl, Br, J) oder CH₂CH₂OH,
- R⁹, R¹⁰: unabhängig voneinander Wasserstoff, Hydroxyl, C₁-C₄-Alkyl, C₁-C₄-Alkoxy, worin Alkyl vorzugsweise für Methyl oder Ethyl steht, Halogen (F, Cl, Br, J), Nitro, Cyano, eine -N=N-N(R²)₂- oder eine OH-Gruppe,
- Y: OH oder N(R¹)₂ und
- R¹: Wasserstoff, Methyl oder Ethyl bedeuten
mit der Maßgabe, dass pro Gesamtmolekül (2) ein bis zwei -N=N-N(R²)₂- und ein bis zwei OH-Gruppen an beliebigen Ring-Kohlenstoffatomen von aromatischen Ringen stehen und dann sulfatieren bzw. sulfoamidieren.

Die erfindungsgemäßen Diphenylderivate werden nachstehend je nach Definition von X im Einzelnen erläutert:

### 1. cis- und trans-Stilbene der Formel (5)

Wenn in Formel (1) X für -CR⁷=CR⁸- steht, werden die erfindungsgemäßen Verbindungen durch die Formel 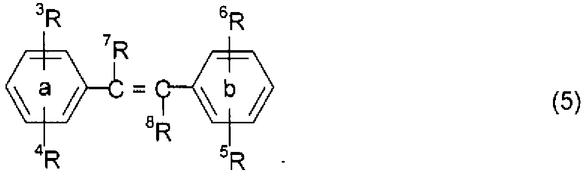 wiedergegeben, worin
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -N=N-N(R²)₂- oder eine -OSO₂Y-Gruppe,
- R⁷: Wasserstoff, Methyl, Ethyl oder einen durch die Reste R⁹, R¹⁰ substituierten Phenylrest, der direkt an das R⁷-tragende C-Atom geknüpft ist,
- R⁸: Wasserstoff, Ethyl, CN, NO₂, -CH₂CH₂-Halogen (F, Cl, Br, J) oder CH₂CH₂OH,,
- R⁹, R¹⁰: unabhängig voneinander Wasserstoff, Hydroxyl, C₁-C₄-Alkyl, C₁-C₄-Alkoxy, worin Alkyl vorzugsweise für Methyl oder Ethyl steht, Halogen (F, Cl, Br, J), Nitro, Cyano, eine -N=N-N(R²)₂- oder eine -OSO₂Y-Gruppe,
- Y: OH oder N(R¹)₂,
- R¹: Wasserstoff, Methyl oder Ethyl und
- R²: Methyl oder Ethyl bedeuten

mit der Maßgabe, dass pro Gesamtmolekül (5) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Bevorzugt sind Verbindungen der Formel (5), worin
- R⁴, R⁵: jeweils eine (CH₃)₂N-N=N-Gruppe in 3- und in 3'- Stellung,
- R³, R⁶: jeweils eine -OSO₂Y-Gruppe in 4- und in 4'- Stellung,
- R⁷, R⁸: jeweils Ethyl und
- Y: OH oder NH₂ bedeuten
mit der Maßgabe, dass pro Gesamtmolekül (5) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Weiterhin sind Verbindungen der Formel (5) bevorzugt, worin
- R⁴, R⁵: unabhängig voneinander Wasserstoff, eine (CH₃)₂N-N=N- oder eine - OSO₂Y-Gruppe, R³, R⁶ unabhängig voneinander Wasserstoff, Fluor, Chlor, Hydroxy, Methyl, Methoxy oder eine -OSO₂Y-Gruppe,
- R⁷, R⁸: jeweils Wasserstoff und
- Y: OH oder NH₂ bedeuten
mit der Maßgabe, dass pro Gesamtmolekül (5) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Besonders bevorzugt sind Verbindungen der Formel (5), worin
- R³: Wasserstoff, Hydroxy oder eine -OSO₂Y-Gruppe in 4-Stellung,
- R⁴: eine -OSO₂Y-Gruppe in 2-Stellung,
- R⁵: eine (CH₃)₂N-N=N-Gruppe in 4'-Stellung,
- R⁶: Wasserstoff oder Methyl in 2'-Stellung,
- R⁷, R⁸: jeweils Wasserstoff und
- Y: OH oder NH₂ bedeuten
mit der Maßgabe, dass pro Gesamtmolekül (5) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Als besonders bevorzugte Stilbene (5) im Rahmen der Erfindung seien die folgende Verbindungen genannt:
(Z+E) 4-(3,3-Dimethyltriazenyl-1)- 4'-natriumsulfooxy-2'-sulfamoyloxy-stilben 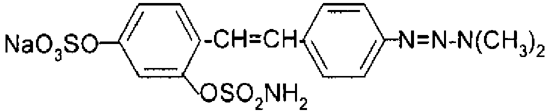
(Z+E) 4-(3,3-Dimethyltriazenyl-1)-4'-hydroxy-2'-natriumsulfooxy-stilben 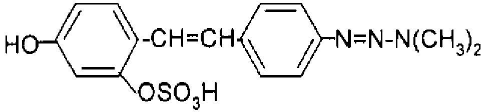
(Z und E) 4-(Dimethyltriazenyl-1)-2-methyl-4'-hydroxy-2'-natriumsulfooxy-stilben 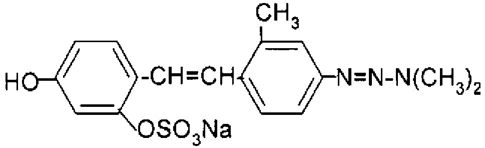
3,3'-[Di-(3,3-dimethyltriazenyl-1)]-4,4'-(di-natriumsulfooxy)-1,2-diethyl-stilben 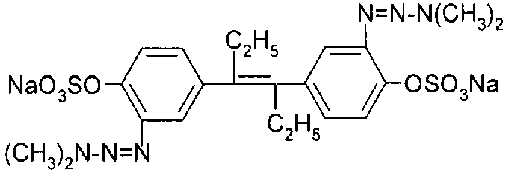 sowie deren Salze, freie Säuren, Solvate und die Solvate dieser Salze und die Solvate dieser freien Säuren.

Die Verbindungen (5) können analog den Verbindungen (1) aus den aromatischen Aminen 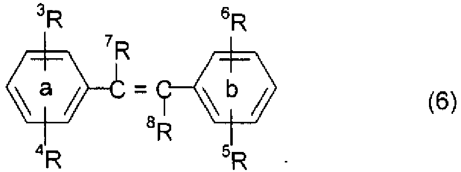 worin
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -NH₂- oder eine -OSO₂Y-Gruppe,
- R⁷: Wasserstoff, Methyl, Ethyl oder einen durch die Reste R⁹, R¹⁰ substituierten Phenylrest, der direkt an das R⁷-tragende C-Atom geknüpft ist,
- R⁸: Wasserstoff, Ethyl, CN, NO², -CH₂CH₂ -Halogen (F, Cl, Br, J) oder CH₂CH₂OH,
- R⁹, R¹⁰: unabhängig voneinander Wasserstoff, Hydroxyl, C₁-C₄-Alkyl, C₁-C₄-Alkoxy, worin Alkyl vorzugsweise für Methyl oder Ethyl steht, Halogen (F, Cl, Br, J), Nitro, Cyano, eine -NH₂- oder eine -OSO₂Y-Gruppe,
- Y: OH oder N(R¹)₂ und
- R¹: Wasserstoff, Methyl oder Ethyl bedeuten
mit der Maßgabe, dass pro Gesamtmolekül (6) ein bis zwei -NH₂- und ein bis zwei -OSO₂Y-Gruppen an beliebigen Ring-Kohlenstoffatomen von aromatischen Ringen stehen, hergestellt werden.

Verwendet man z. B. 3, 3' Di-amino-4,4'-dikaliumsulfooxy-1,2-diethyl-stilben, Kaliumnitrit und Dimethylamin als Ausgangsstoffe, so kann der Reaktionsverlauf durch das Formelschema 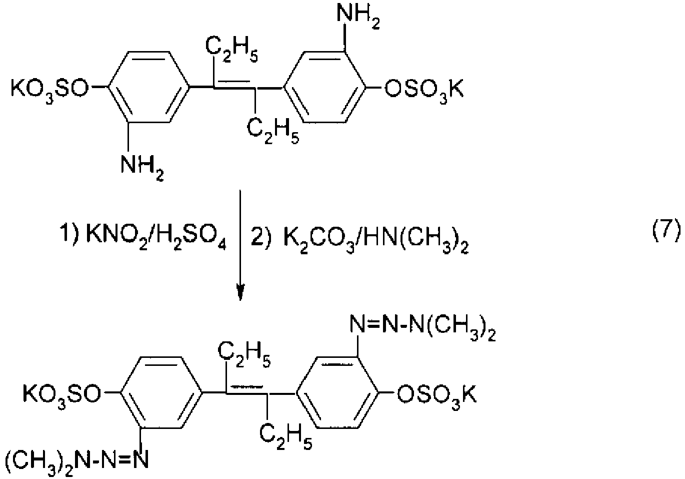 dargestellt werden.

Das im Formelschema (7) genannte 3,3' Diamino-4,4'-dikaliumsulfooxy-1,2-diethyl-stilben kann aus dem bekannten 3,3'Dinitro-diethylstilbestrol (Arch. Pharm. (Weinheim) 311, 184-195 (1978)) mit sich anschschließender Sulfatierung und Reduktion zum Diamin umgewandelt werden.

Die aromatischen Amine (6), können aus den entsprechenden Nitro-sulfooxy- bzw. -sulfamoyloxyaromaten in an sich bekannter Weise z.B. durch katalytische Reduktion mit Wasserstoff an Palladium/Kohle in Ethanol oder an Platinoxyd in Ethanol (Chem. Berichte. 72, 839, [(1939)]) oder mit Raney-Nickel in THF (Chem. Berichte. 91, 1905 (1958)) hergestellt werden.
Die hier genannten Syntheseverfahren können auch zur Synthese der in den Abschnitten 2-4 verwendeten aromatischen Amine genutzt werden.

Beispiele für die Umsetzung aromatischer Amine zu Diazoniumsalzen und deren Kupplung mit Dialkylaminen zu (3,3-Dialkyltriazenyl-1)-arylen sind ausführlich in DE 17 93 115 A1, DE 21 47 781 A1, GB 1 371 969 und WO 2004/106358 beschrieben.

Die Herstellung der neuen Ausgangsprodukte mit Sulfooxy- bzw. Sulfamoyloxygruppen kann durch die Umsetzung der entsprechenden Phenolaromaten mit Alkalidisulfat (Na, K) gemäß EP 0 722 455 A1/US 5,703,261, gegebenenfalls alternativ auch mit Chlorsulfonsäure/Pyridin gemäß WO 02/134715 A1 bzw. mit Sulfamoylchloriden in Dimethylacetamid gemäß WO 2008/003378 und
US 2003/0225051, Seite 44 erfolgen.
Die hier genannten Synthesen können auch in den Reaktionen nach Abschnitten 2-4 bei der Umsetzung phenolischer Hydroxylgruppen zu den erfindungsgemäßen Ausgangs- bzw. Endprodukten mit Sulfooxy- bzw. Sulfamoyloxygruppen verwendet werden.

Die Synthese der erfindungsgemäßen Verbindungen kann durch die Reaktionsschemata 8 und 9 erläutert werden:

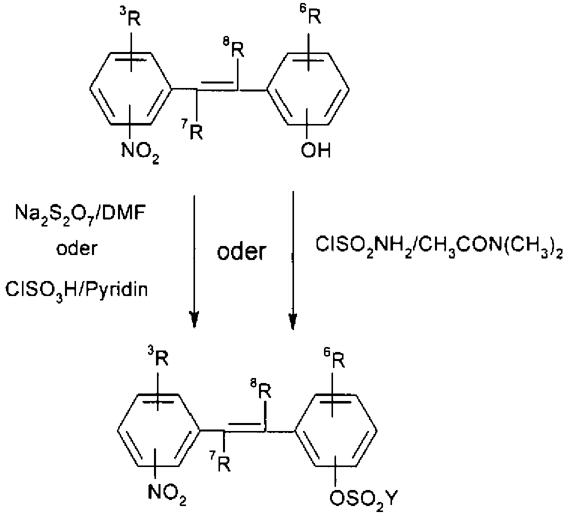

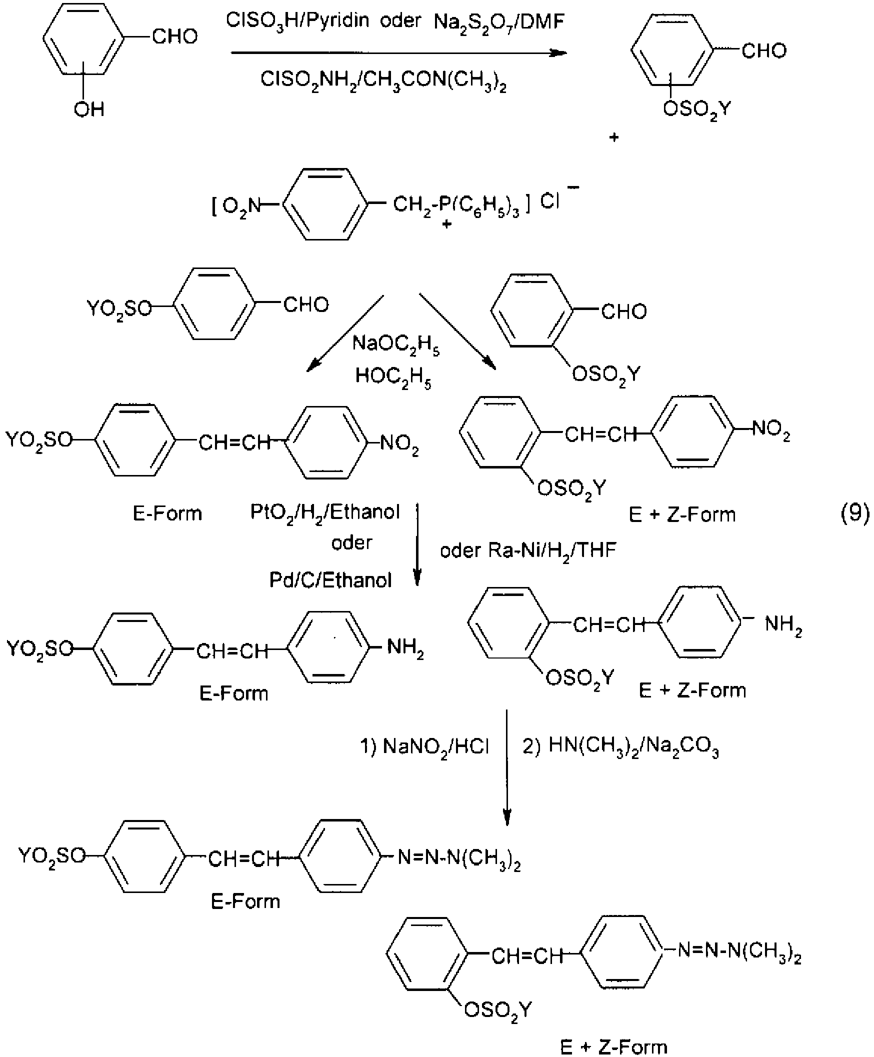

### 2. Triphenylethylen-derivate der Formel (10)

Wenn in Formel (1)
- X: -CR⁷=CR⁸- und R⁷ einen durch R⁹, R¹⁰ substituierten Phenylrest, der direkt an das R⁷-tragende C-Atom geknüpft ist, bedeuten, werden diese Verbindungen durch die Formel 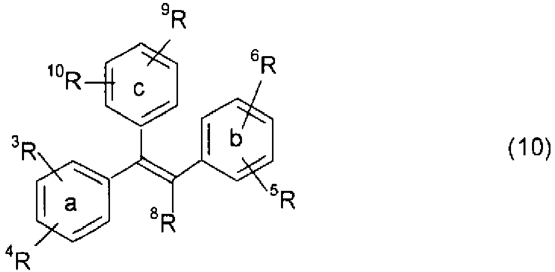
wiedergegeben, worin
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -N=N-N(R²)₂- oder eine -OSO₂Y-Gruppe,
- R⁸: Wasserstoff, Ethyl, CN, NO₂,,-CH₂-CH₂-Halogen (F, Cl, Br, J) oder,CH₂CH₂OH,
- R⁹, R¹⁰: unabhängig voneinander Wasserstoff, Hydroxyl, C₁-C₄-Alkyl, C₁-C₄-Alkoxy, worin Alkyl vorzugsweise für Methyl oder Ethyl steht, Halogen (F, Cl, Br, J), Nitro, Cyano, eine -N=N-N(R²)₂- oder eine -OSO₂Y-Gruppe,
- Y: OH oder N(R¹)₂,
- R¹: Wasserstoff, Methyl oder Ethyl und
- R²: Methyl oder Ethyl bedeuten
mit der Maßgabe, dass pro Gesamtmolekül (10) ein bis zwei -N=N-N(R²)₂- und ein bis zwei -OSO₂Y-Gruppen an beliebigen Ring-Kohlenstoffatomen von aromatischen Ringen stehen,

sowie deren Salze, freie Säuren, Solvate und die Solvate dieser Salze und die Solvate dieser freien Säuren.

Bevorzugt sind Verbindungen der Formel (10), worin
- R³: Wasserstoff, Hydroxy, Methoxy, eine -OSO₂Y-Gruppe, Halogen (F, Cl, Br, J), CHO oder CH₂OH in 3- oder in 4- Stellung,
- R⁴: Wasserstoff oder eine -N=N-N(CH₃)₂-Gruppe in 3-Stellung,
- R⁵: Wasserstoff oder eine -N=N-N(CH₃)₂-Gruppe in 3'- oder in 4'- Stellung,
- R⁶: Wasserstoff, Hydroxy, Methoxy oder eine -OSO₂Y-Gruppe in 3'- oder in 4'-Stellung,
- R⁸: Wasserstoff, Ethyl, CH₂CH₂Halogen (F, Cl) oder CH₂CH₂OH,
- R⁹, R¹⁰: unabhängig voneinander Hydroxy, Methoxy oder eine -OSO₂Y-Gruppe in 3"- oder in 4"-Stellung und
- Y: OH oder NH₂ bedeuten
mit der Maßgabe, dass pro Gesamtmolekül (10) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Besonders bevorzugt sind Verbindungen der Formel 10, worin
- R³: Wasserstoff, Hydroxy, Methoxy oder eine -OSO₂Y-Gruppe in 4-Stellung,
- R⁴: Wasserstoff oder eine -N=N-N(CH₃)₂-Gruppe in 3-Stellung,
- R⁵: Wasserstoff oder eine -N=N-N(CH₃)₂-Gruppe in 3'- oder in 4'- Stellung,
- R⁶: Wasserstoff, Hydroxy, Methoxy oder eine -OSO₂Y-Gruppe in 4'-Stellung,
- R⁸: Wasserstoff oder Ethyl,
- R⁹, R¹⁰: unabhängig voneinander Hydroxy, Methoxy oder eine -OSO₂Y-Gruppe in 3"- oder in 4"-Stellung und
- Y: OH oder NH₂ bedeuten
mit der Maßgabe, dass pro Gesamtmolekül (10) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und Solvate dieser Salze und Solvate dieser freien Säuren.

Als besonders bevorzugte Triphenylethylen-derivate (10) seien die folgende Verbindungen genannt:
1,1-[Bis-3,4"-(di-natriumsulfooxy-phenyl)]-2-ethyl-2-[4'-(3,3-dimethyltriazeno-1)-phenyl]-ethen 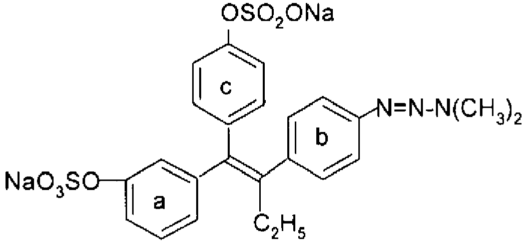
1-(4-Hydroxy-phenyl)-1-(4"-(natriumsulfooxy-phenyl)-2-ethyl-2-[4'-(3,3-dimethyltriazenyl-1)-phenyl]-ethen 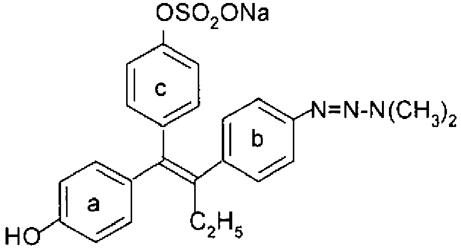
1-[3-(3,3-Dimethyltriazenyl-1)-4-methoxy-phenyl]-1-(4"-natriumsulfooxy-phenyl)-2-ethyl-2-(4'-hydroxy-phenyl)-ethen 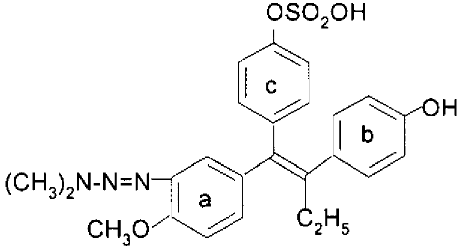
1,1-[Bis-4-(natriumsulfooxy-phenyl)]-2-ethyl-2-[4'-(3,3-dimethyltriazenyl-1)-phenyl]-ethen 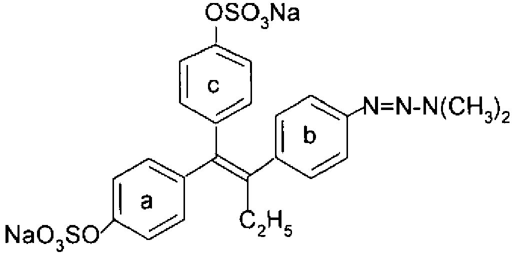 sowie deren Salze, freie Säuren, Solvate und die Solvate dieser Salze und die Solvate dieser freien Säuren.

Die Verbindungen (10) können analog den Verbindungen (1) aus den entsprechenden aromatischen Aminen 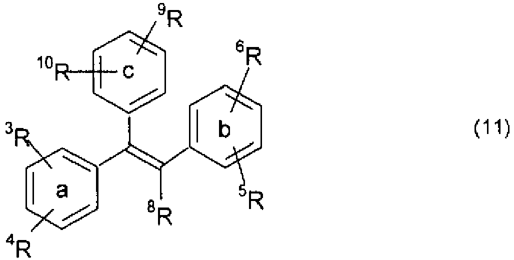 hergestellt werden, worin
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -NH₂- oder eine -OSO₂Y-Gruppe,
- R⁸: Wasserstoff, Ethyl, CN, NO₂, -CH₂CH₂-Halogen (F, Cl, Br, J) oder CH₂CH₂OH,
- R⁹, R¹⁰: unabhängig voneinander Wasserstoff, Hydroxyl, C₁-C₄-Alkyl, C₁-C₄-Alkoxy, worin Alkyl vorzugsweise für Methyl oder Ethyl steht, Halogen (F, Cl, Br, J), Nitro, Cyano, eine -NH₂- oder eine -OSO₂Y-Gruppe,
- Y: OH oder N(R¹)₂ und
- R¹: Wasserstoff, Methyl oder Ethyl bedeuten
mit der Maßgabe, dass pro Gesamtmolekül (11) ein bis zwei -NH₂- und ein bis zwei -OSO₂Y-Gruppen an beliebigen Ring-Kohlenstoffatomen von aromatischen Ringen stehen.

Verwendet man z. B. 1-(4-Aminophenyl)-1-(4"-natriumsulfooxy-phenyl)-2-ethyl-2-phenyl-ethen, Natriumnitrit und Dimethylamin als Ausgangsstoffe, so kann der Reaktionsverlauf durch das folgende Formelschema 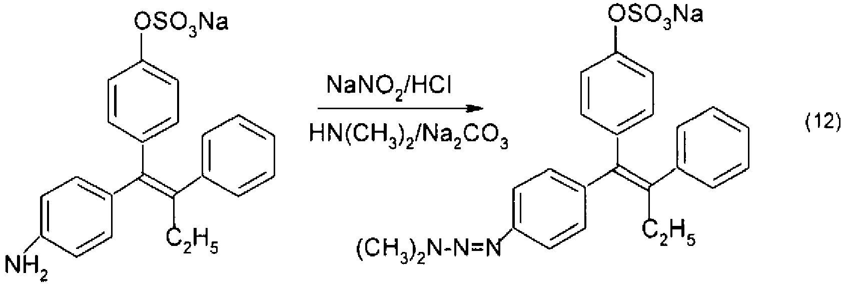 dargestellt werden.

Dabei wird im ersten Schritt ein Diazoniumsalz hergestellt, welches sofort in einem zweiten Schritt mit Dimethylamin zum 1-(4-(3,3-Dimethyltriazeno-1-phenyl)-1-(4"-natriumsulfooxy-phenyl)-2-ethyl-2-phenyl-ethen umgesetzt werden kann.

Die aromatischen Amine (11) können aus den entsprechenden Nitro-sulfooxy- bzw. -sulfamoyloxyaromaten nach allgemein bekannten Methoden, z. B. durch katalytische Reduktion mit Wasserstoff an Palladium/Kohle in Ethanol oder mit Ra-Ni in THF hergestellt werden.

Die Triphenylethylen-derivate der Formel (10) können auf dem im Formelschema (13) gezeigten Weg hergestellt werden. Dabei verläuft die Synthese nach bekannten Methoden wie die Wittig-Reaktion, die Reduktion der Nitrogruppe mit Raney-Nickel/Hydrazin, die Etherspaltung, die Herstellung des Triazenylphenols und seine Umsetzung mit Alkalidisulfat bzw. Sulfamoylchlorid. Anstelle von Alkalidisulfat in DMF kann auch Chlorsulfonsäure in Pyridin verwendet werden.

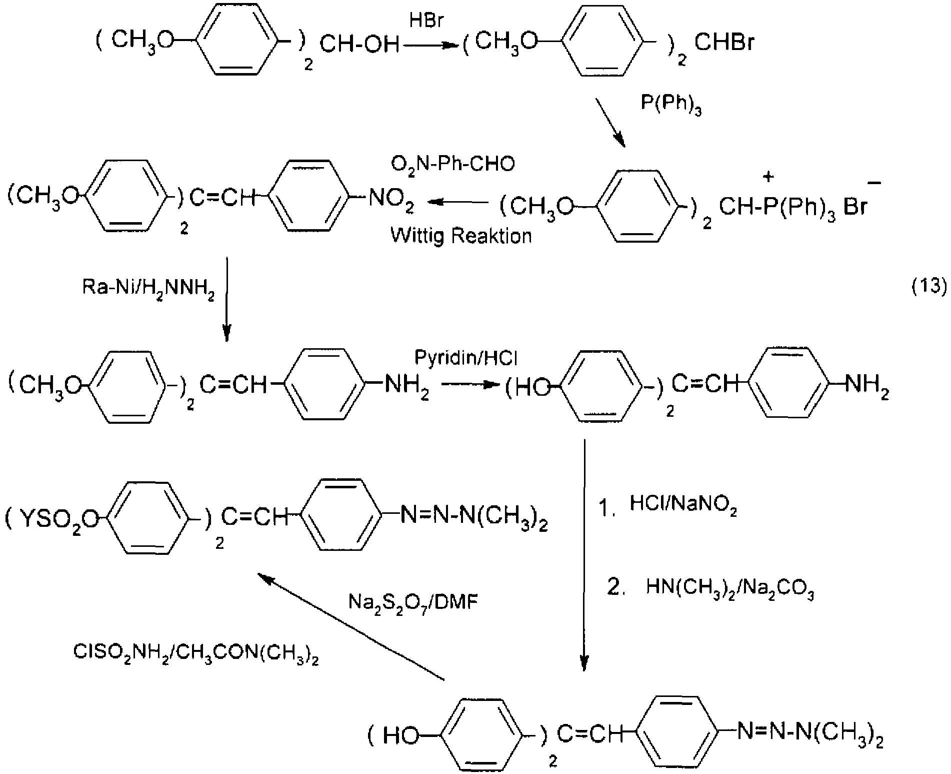

### 3. Isoflavone der Formel (14) (nicht gemäß der Erfindung)

Wenn in Formel (1)
- X: einen zweibindigen -C(O)-C*=CH-O- Rest, der zusammen mit den zwei benachbarten C-Atomen des Ringes a, an dem er steht, einen Pyranonring bildet, wobei der Ring b am C*-Atom dieses Restes steht, nimmt Formel (1) die Gestalt der Formel 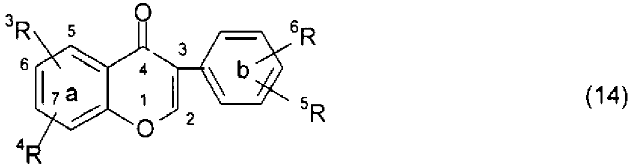
an, worin
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -N=N-N(R²)₂- oder eine -OSO₂Y-Gruppe,
- Y: OH oder N(R¹)₂,
- R¹: Wasserstoff, Methyl oder Ethyl und
- R²: Methyl oder Ethyl bedeuten
mit der Maßgabe, dass pro Gesamtmolekül (14) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Bevorzugt sind Verbindungen der Formel (14), worin
- R³, R⁶: unabhängig voneinander Hydroxy, Methoxy oder eine -OSO₂Y-Gruppe,
- R⁴, R⁵: unabhängig voneinander Wasserstoff, Hydroxy, Methoxy oder eine -N=N-N(CH₃)₂-Gruppe und
- Y: OH oder NH₂ bedeuten
mit der Maßgabe, dass pro Gesamtmolekül (14) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Besonders bevorzugt sind Verbindungen der Formel (14), worin
- R³: Hydroxy, Methoxy oder eine -OSO₂Y-Gruppe in 5- oder in 7- Stellung,
- R⁴: Wasserstoff oder eine -N=N-N(CH₃)₂-Gruppe in 6- Stellung,
- R⁵: Hydroxy, Methoxy oder eine -OSO₂Y-Gruppe in 4'-Stellung,
- R⁶: eine -N=N-N(CH₃)₂-Gruppe in 2'- oder in 3'- Stellung und
- Y: OH oder NH₂ bedeuten
mit der Maßgabe, dass pro Gesamtmolekül (14) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Als besonders bevorzugte Isoflavone (14) seien die folgende Verbindungen genannt:
7-Hydroxy-3-[3'-(3,3-dimethyltriazenyl-1)-4'-natriumsulfooxy]-isoflavon 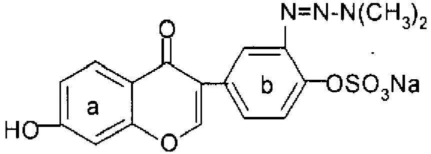
6-(3,3-Dimethyltriazenyl-1)-7-methoxy-3-(4'-natriumsulfooxy)-isoflavon 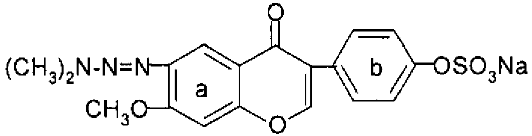
7-Methoxy-3'-(3,3-dimethyltriazenyl-1)-4'-natriumsulfooxy-isoflavon 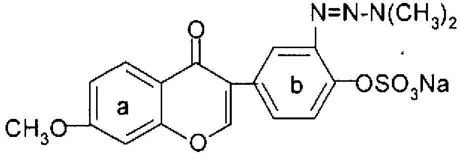
7-Natriumsulfooxy-2'-(3,3-dimethyltriazenyl-1)-4'-sulfamoyloxy-isoflavon 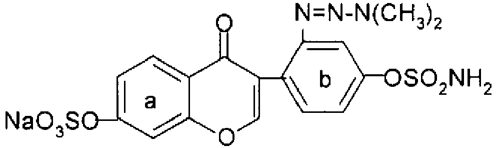 sowie deren Salze, freie Säuren, Solvate und die Solvate dieser Salze und die Solvate dieser freien Säuren.

Die nicht erfindungsgemäßen Isoflavone (3) können analog den Verbindungen (1) aus den entsprechenden aromatischen Aminen 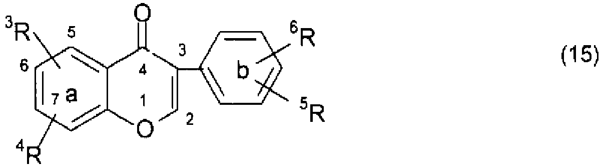 worin
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -NH₂- oder eine -OSO₂Y-Gruppe,
- Y: OH oder N(R¹)₂ und
- R¹: Wasserstoff, Methyl oder Ethyl bedeuten
mit der Maßgabe, dass pro Gesamtmolekül (15) ein bis zwei -NH₂- und ein bis zwei -OSO₂Y-Gruppen an beliebigen Ring-Kohlenstoffatomen von aromatischen Ringen stehen, hergestellt werden.

Verwendet man z. B. 6-Amino-7,4'-dikaliumsulfooxy-isoflavon, Natriumnitrit und Dimethylamin als Ausgangsstoffe, so kann der Reaktionsverlauf durch das folgende Formelschema wiedergegeben werden.

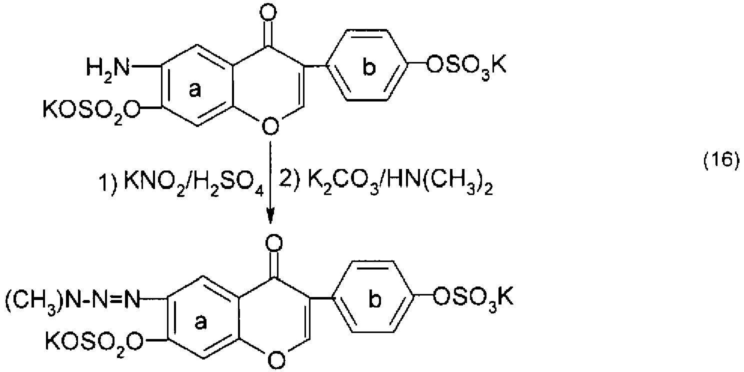

Dabei wird im ersten Schritt ein Diazoniumsalz hergestellt, welches in einem zweiten Schritt sofort mit Dimethylamin das erfindungsgemäße 7,4'-Dikaliumsulfooxy-6-(3,3-dimethyltriazenyl-1)-isoflavan bildet.

Die bislang nicht bekannten aromatischen Amine (15) können aus den entsprechenden Nitro-sulfooxy- bzw. -sulfamoyloxyaromaten nach jedem Fachmann bekannten Methoden, z. B. durch katalytische Reduktion mit Wasserstoff an Palladium/Kohle in Ethanol hergestellt werden oder Ra-Ni in THF.

[0]Die als Ausgangsprodukt verwendeten Nitro-isoflavone des im folgenden Formelschema (17) verwendeten Typs sind bekannt und können nach bekannten Verfahren (Z. Kristallogr. NCS 222, 293-294 (2007)) hergestellt werden.

Die Herstellung der neuen Ausgangsprodukte mit Sulfooxy- bzw.- Sulfamoyloxygruppen kann durch die Umsetzung der entsprechenden Phenolaromaten mit Chlorsulfonsäure in Pyridin oder mit Alkalidisulfat in DMF bzw. mit Sulfamoylchloriden in Dimethylacetamid erfolgen. Die einzelnen Reaktionsschritte werden im Reaktionsschema (17) erläutert:

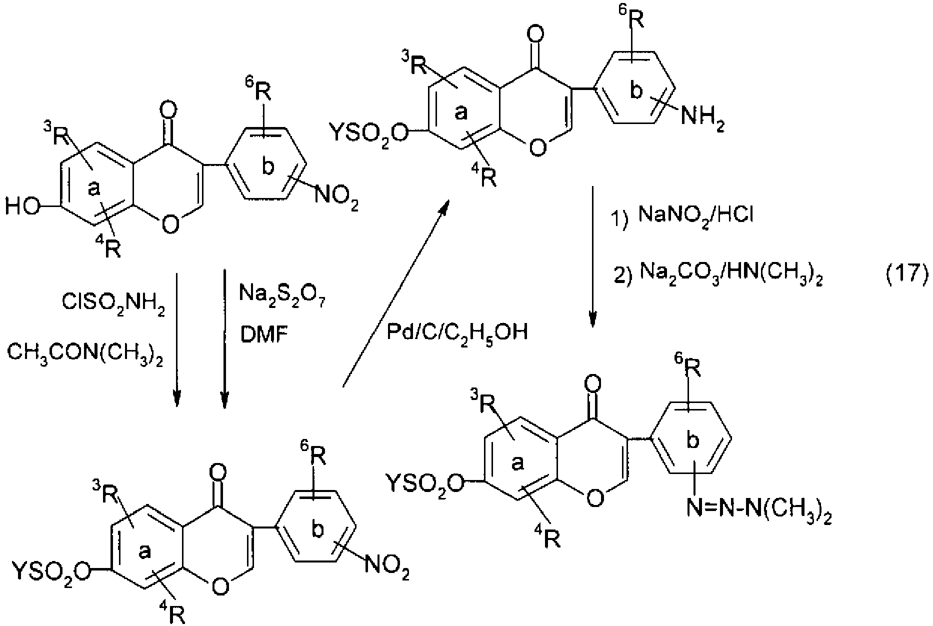

### 4. Unverbrückte oder mit Atomen oder zweiatomigen Gruppen verbrückte

Diphenylderivate der Formel (18) (nicht gemäß der Erfindung, mit der Ausnahme von den Verbindungen, worin X eine CO-Gruppe bedeutet): Formel (1) kann auch die Gestalt von Formel 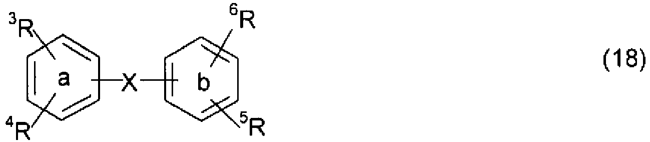 annehmen, worin
- X: eine direkte C-C-Bindung, CH₂, CHOH, CO, S, SO, SO₂ oder -N=N-,
- R³, R⁶: unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, worin Alkyl vorzugsweise Methyl oder Ethyl bedeutet, Halogen (F, Cl, Br, J), Nitro, Cyano oder eine -OSO₂Y-Gruppe,
- R⁴, R⁵: unabhängig voneinander eine -N=N-N(R²)₂- oder eine -OSO₂Y-Gruppe,
- Y: OH oder N(R¹)₂,
- R¹: Wasserstoff, Methyl oder Ethyl und
- R²: Methyl oder Ethyl bedeuten
mit der Maßgabe, dass pro Gesamtmolekül (18) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Bevorzugt sind Verbindungen der Formel (18), worin
- X: CH₂, CO, SO oder SO₂,
- R³, R⁶: unabhängig voneinander Wasserstoff, Hydroxy, Methyl, Ethyl, Methoxy, CH₃S-, CH₃SO-, CH₃SO₂-, Halogen (F, Cl, Br, J), Nitro, Cyano oder die -OSO₂Y-Gruppe,
- R⁴, R⁵: unabhängig voneinander eine -N=N-N(CH₃)₂ - oder eine -OSO₂Y-Gruppe und
- Y: OH oder NH₂ bedeuten
mit der Maßgabe, dass pro Gesamtmolekül (18) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Besonders bevorzugt sind Verbindungen der Formel (18), worin
- X: CH₂, CO, SO oder SO₂,
- R³, R⁶: unabhängig voneinander Wasserstoff, Hydroxy, Methyl, Fluor, Chlor, Brom oder eine -OSO₂Y-Gruppe,
- R⁴: Wasserstoff ,
- R⁵: eine (CH₃)₂N-N=N-Gruppe und
- Y: OH oder NH₂ bedeuten
mit der Maßgabe, dass pro Gesamtmolekül (18) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Ganz besonders bevorzugt sind Verbindungen der Formel (18), worin
- X: CH₂, CO, SO oder SO₂,
- R³: Hydroxy, Methyl, Chlor oder Brom in 2-, 3- oder 4-Stellung,
- R⁴: die -OSO₂Y-Gruppe in 2-, 3- oder 4-Stellung,
- R⁵: eine (CH₃)₂N-N=N-Gruppe in 4'-Stellung.
- R⁶: Wasserstoff, Methyl, Fluor, Chlor oder Brom in 2'-, 3'- oder 5'-Stellung und
- Y: OH oder NH₂ bedeuten
mit der Maßgabe, dass pro Gesamtmolekül (18) ein bis zwei -N=N-N(CH₃)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

Die bevorzugtesten mit zweiatomigen oder dreiatomigen Gruppen verbrückten Diphenylderivate umfassen folgende Verbindungen:
4-(3,3-Dimethyl-triazenyl-1)-4'-hydroxy-2'-natriumsulfooxy-benzophenon 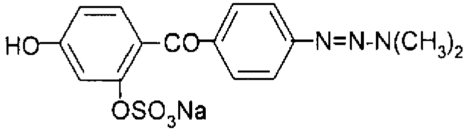
4-(3,3-Dimethyl-triazenyl-1)-4'-hydroxy-3'-natriumsulfooxy-benzophenon 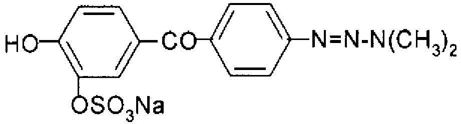
4-(3,3-Dimethyl-triazenyl-1)- 3'-hydroxy-4'-natriumsulfooxy-benzophenon 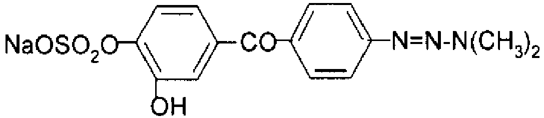
4-(3,3-Dimethyl-triazenyl-1)-4'-natriumsulfoxy-3'-sulfamoyloxy-benzophenon 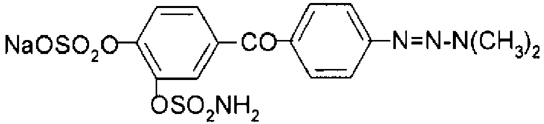
4-(3,3-Dimethyl-triazenyl-1)-4'-natriumsulfooxy-diphenylmethan 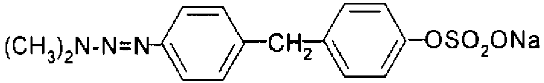
2-Chlor-4-(3,3-dimethyltriazenyl-1)-4'-natriumsulfooxy-diphenylsulfon 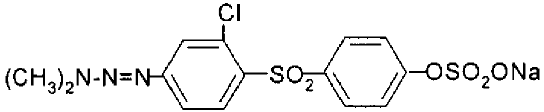
sowie deren Salze, freie Säuren, Solvate und die Solvate dieser Salze und die Solvate dieser freien Säuren.

Im Besondern bevorzugt ist
4-(3,3-dimethyl-triazenyl-1)-4'-natriumsulfooxy-benzophenon

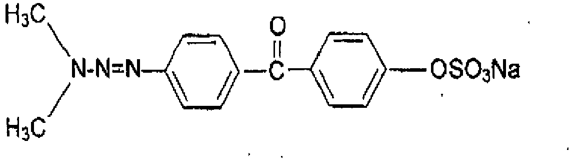

Die Ausgangsprodukte für die Synthese der Verbindungen (18) sind bekannt und können nach den in der GB 1 371 969 beschriebenen Verfahren hergestellt und zur Synthese der Verbindungen (18) genutzt werden, indem die dort beschriebenen Hydroxy-triazenderivate vom Typ (19) zu Endprodukten, z. B. (20), umgesetzt werden, wie im Formelschema (21) exemplarisch dargestellt wird. 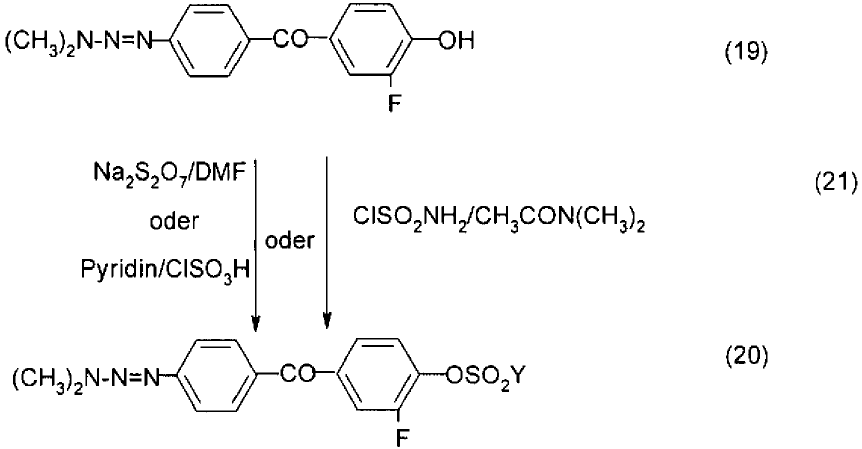 worin Y die in der Legende der Formel (18) angegebene Bedeutung besitzt.

Die vorliegende Erfindung betrifft weiterhin Diphenylderivate, die pro Molekül (i) mindestens eine Dialkyltriazenyl- und (ii) mindestens eine Sulfooxy- und/oder mindestens eine Sulfamoyloxygruppe enthalten, und deren Salze, Solvate und Solvate dieser Salze zur Verwendung als Arzneimittel sowie die Verwendung dieser Verbindungen zur Herstellung von Arzneimitteln, vorzugsweise zur Behandlung von Krebserkrankungen der von Estrogenen abhängigen Gewebe, wie insbesondere von Estrogenrezeptor-negativen und Estrogenrezeptor-positiven Tumoren der Geschlechtsorgane und des Melanoms.

In einer bevorzugten Ausführungsform der vorliegenden Erfindung können die erfindungsgemäßen substituierten Diphenylderivate in Kombination mit monoklonalen Antikörpern eingesetzt werden.

Gemäß der vorliegenden Erfindung werden bevorzugt monoklonale Antikörper eingesetzt, die eine hohe Selektivität in der Tumorzelle haben (Discovery Medicine, Alain Beck, "The next generation of Antibody-drug Conjugates Comes of Age", 16. Oktober 2010).

Die erfindungsgemäßen substituierten Diphenylderivate werden an den monoklonalen Antikörper gebunden und in die Tumorzelle eingeführt.

Im allgemeinen werden die erfindungsgemäßen Diphenylderivate und die monoklonalen Antikörper im Verhältnis 0,5 zu 1 (Diphenylderivat zu Antikörper) bis 4 zu 1 eingesetzt.

Monoklonale Antikörper sind an sich bekannt. Monoklonale Antikörper sind immunologisch aktive Proteine, die von einer auf einen einzigen B-Lymphozyten zurückgehenden Zelllinie produziert werden und die sich gegen ein einzelnes Epitop (z. B. einer Tumorzelle) richten.

Die erfindungsgemäßen Verbindungen können beispielsweise zur Behandlung folgender Tumorarten verwendet werden: Brustkrebs (Mammakarzinom), Gebärmutterkrebs (Endometriumkarzinom, Corpuskarzinom), Gebärmutterhalskrebs, Eierstockkrebs (Ovarialkarzinom), Scheidenkrebs, Prostatakrebs, Hautkrebs, Melanom (malignes Melanom).

Die Erfindung betrifft weiterhin die Verwendung der erfindungsgemäßen Verbindungen in Kombination mit mindestens einem weiteren Chemotherapeutikum zur Behandlung von Krebs. Die erfindungsgemäßen Verbindungen können demnach auch in Kombination mit weiteren in der Behandlung von Krebs bzw. Tumoren bekannten Chemotherapeutika und/ oder mit Arzneimitteln, die begleitend mit den Chemotherapeutika während einer Chemotherapie verabreicht werden, verwendet werden.

Beispiele solcher in Kombination anwendbarer Chemotherapeutika und weiterer in der Chemotherapie eingesetzter Arzneimittel finden sich beispielsweise in der WO/2007/061978 unter dem Stichwort "Combination Therapy" (Seite 23, Zeile 1 bis Seite 30, Zeile 18) oder in der US 2007/135424 A1 (Abschnitte 153 bis 171).

Für diese Applikationswege kann der Wirkstoff in geeigneten Applikationsformen verabreicht werden. Für die orale Applikation eignen sich bekannte, den Wirkstoff schnell und/oder modifiziert abgebende Applikationsformen, wie z. B. Tabletten (nicht überzogene sowie überzogene Tabletten, z. B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan oder intraperitoneal). Für die parenterale Applikation eignen sich als Applikationsformen u. a. Injektions- und Infusionszubereitungen in Form von Lösungen, Suspensionen, Emulsionen, Lyophilisaten und sterilen Pulvern.

Für die sonstigen Applikationswege eignen sich z. B. Inhalationsarzneiformen (u. a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies kann unter Verwendung inerter nichttoxischer, pharmazeutisch geeigneter Hilfsstoffe geschehen. Hierzu zählen u. a. Trägerstoffe (z. B. mikrokristalline Cellulose), Lösungsmittel (z. B. flüssige Polyethylenglykole), Emulgatoren (z. B. Natriumdodecylsulfat), Dispergiermittel (z. B. Polyvinylpyrrolidon), synthetische und natürliche Biopolymere (z. B. Albumin), Stabilisatoren (z. B. Antioxidantien wie Ascorbinsäure), Farbstoffe (z. B. anorganische Pigmente wie Eisenoxide) oder Geschmacks- und/oder Geruchsstoffe.

Im allgemeinen ist zu empfehlen, bei parenteraler Applikation Mengen von etwa 1 bis 40 mg/kg, vorzugsweise etwa 2,5 bis 15 mg/kg, Körpergewicht zur Erzielung wirksamer Ergebnisse zu verabreichen. Bei oraler Applikation beträgt die Menge etwa 1 bis 70 mg/kg, vorzugsweise etwa 1 bis 50 mg/kg, Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte Obergrenze überschritten werden muss. Im Falle der Applikation größerer Mengen kann es empfehlenswert sein, diese in mehreren Einzelgaben über den Tag zu verteilen.

Die vorliegende Erfindung betrifft weiterhin pharmazeutische Zusammensetzungen, enthaltend mindestens eine der erfindungsgemäßen Verbindungen zusammen mit mindest einem pharmakologisch verträglichen Träger, Hilfsstoff oder Lösungsmittel. Dabei handelt es sich um übliche pharmazeutische Träger, Hilfsstoffe oder Lösungsmittel. Die erfindungsgemäßen pharmazeutische Zusammensetzungen sind beispielsweise geeignet zur Inhalation oder zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Inhalationspulvern, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulver, Inhalationspulvern, mikrokristallinen Formulierungen, Inhalationssprays, Puder, Tropfen, Nasentropfen, Nasensprays, Aerosolen, Ampullen, Lösungen, Säfte, Suspensionen, Emulsionen, Infusionslösungen oder Injektionslösungen etc. vor.

### Beispiele:

Die Prozentangaben in den folgenden Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### 1. Stilbenderivate der Formel (5)

### Ausgangsprodukte

### Beispiel 1.1: 2-Natriumsulfooxy-benzaldehyd

Die nachstehende Vorschrift kann als Mustervorschrift zur Sulfatierung mit Alkalidisulfaten dienen

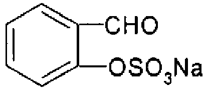

12,2 g (0,1 Mol) 2-Hydroxybenzaldehyd und 33,3 g (0,15 Mol) Natriumdisulfat werden mit 100 ml Dimethylformamid und 130 ml Tetrahydrofuran versetzt und 30 Stunden lang bei Raumtemperatur gerührt. Dann setzt man der Reaktionsmischung 200 ml einer 1 n wässrigen Natriumcarbonatlösung zu, verrührt die Mischung, filtriert und engt das Filtrat unter vermindertem Druck ein. Der Rückstand wird in der gerade nötigen Menge Wasser gelöst, über Aktivkohle filtriert und das Filtrat am Rotationsverdampfer eingeengt.

Die Titelsubstanz kann als Rohprodukt weiterverarbeitet werden.

### Beispiel 1.2: 4-Natriumsulfooxy-benzaldehyd

Die Verbindung wird analog Beispiel 1.1 hergestellt und das Rohprodukt weiterverarbeitet.

Analog dazu sind die 3- Natriumsulfooxy-benzaldehyde erhältlich.

### Beispiel 1.3: 2-Sulfamoyloxy-benzaldehyd

Die nachstehende Vorschrift dient als Mustervorschrift zur Sulfamylierung mit Sulfamoylchlorid,

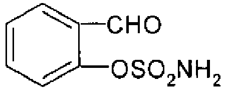

Zu einer Lösung von 12,2 g (0,1 Mol) 2-Hydroxybenzaldehyd in 200 ml wasserfreiem Dimethylacetamid werden bei 0 bis 5°C 12,46 g (0,11 Mol) Sulfamoylchlorid getropft. Das Reaktionsgemisch wird 3 Stunden bei Raumtemperatur gerührt und dann mit 1000 ml Essigsäureethylester extrahiert, die organische Phase mit einer gesättigten wässrigen Natriumhydrogencarbonatlösung gewaschen, über Natriumsulfat getrocknet und das Lösungsmittel am Rotationsverdampfer abgezogen. Der Rückstand wird mit einem Gemisch aus Essigsäureethylester/ Petrolether verrieben, zur Kristallisation gebracht und abgesaugt.

### Beispiel 1.4: 4-Sulfamoyloxy-benzaldehyd

### Synthese analog Beispiel 1.3

Analog sind die 3-Sulfamoyloxy-derivate des 3-Hydroxy-benzaldehyds erhältlich.

### Beispiel 1.5: (4-Nitrobenzyl)-triphenylphosphonium-chlorid

[O₂N-C₆H₄-CH₂-P⁺Ph₃]Cl⁻

Eine Lösung aus 263 g (1 Mol) Triphenylphosphin und 172 g (1 Mol) 4-Nitrobenzylchlorid in 21 Toluol wird 15 Stunden bei Siedetemperatur gerührt. Die Reaktionsmischung wird abgekühlt, das Kristallisat abgesaugt und mit Toluol gewaschen.

### Beispiel 1.6: (Z- und E)-2-Natriumsulfooxy-4'-nitro-stilben.

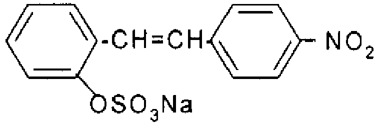

Zu einer Lösung von 8,9 g (0,04 Mol) Natrium-benzaldehyd-2-sulfat in 50 ml Methanol werden gleichzeitig in kleinen Portionen bei 0°C unter Rühren 0,04 Mol Natriummethylat (in methanolischer Lösung) und eine Lösung aus 17,3 g Phosphoniumsalz (aus Beispiel 1.5) in 40 ml Methanol getropft. Nach Entfärbung der Reaktionslösung wird das Lösungsmittel am Rotationsverdampfer abdestilliert. Das Festprodukt wird mit 100 ml Essigsäureethylester zwecks Entfernung des Triphenylphosphinoxids unter Rühren extrahiert, abgesaugt, an der Luft getrocknet, danach mit Nitromethan aufgekocht und vom unlöslichen Rückstand (trans-Verbindung) abgesaugt. Beim Abkühlen kristallisiert aus dem Filtrat die cis-Verbindung aus, die ggf. nochmals umkristallisiert wird.

### Beispiel 1.7: (Z- und E)-4-Methoxy-2-natriumsulfooxy-4'-nitro-stilben

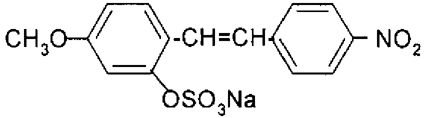

### Synthese analog Beispiel 1.6

### Beispiel 1.8: (Z- und E)- 4-Nitro-2'-sulfamoyloxy-stilben

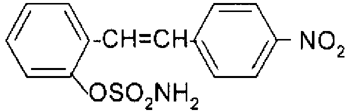

Bei 0 bis 5°C werden gleichzeitig 13,15 g (0,05 Mol) Phosphoniumsalz aus Beispiel 1.5 und 0,05 Mol Natriummethylatlösung portionsweise (nach jeweiliger Entfärbung) zu einer Lösung aus 10,4 g (0,05 Mol) Aldehyd aus Beispiel 1.3 und 75 ml Ethanol gegeben. Nach Entfärbung der Reaktionslösung wird das Lösungsmittel am Rotationsverdampfer abgezogen und der Rückstand mit 50 ml Toluol zur Entfernung des Triphenylphosphins gerührt und abgesaugt. Der verbleibende Rückstand wird mit 75 ml Acetonitril erhitzt und heiß abgesaugt. Zurück bleibt die trans-Verbindung. Die cis-Verbindung kristallisiert aus Acetonitril in der Kälte aus und kann ggf. noch aus Acetonitril oder Isopropanol umkristallisiert werden.

### Beispiel 1.9: (Z)-4-Amino-(2-natriumsulfooxy)-stilben

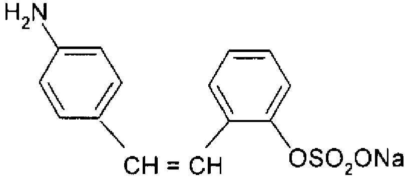

3,4 g (0,01 Mol) Z-Nitroverbindung des Beispiels 1.8 werden in 300 ml Methanol oder THF und 20 ml Wasser mit 4 g Ra-Ni bei Raumtemperatur und 2 atm hydriert. Nach der Aufnahme der nötigen Menge Wasserstoff wird der Katalysator abgesaugt und das Lösungsmittel abdestilliert. Der Rückstand wird als Rohprodukt weiter umgesetzt.

Analog werden die folgenden Aminoverbindungen erhalten:

### Beispiel 1.10: (E)- 4-Amino-2'-natriumsulfooxy-stilben (aus Beispiel 1.6 E)

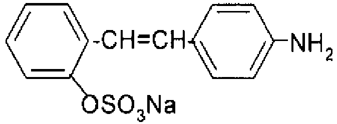

### Beispiel 1.11: (Z)- 4-Amino-2'-sulfamoyloxy-stilben (aus Beispiel 1.8 Z)

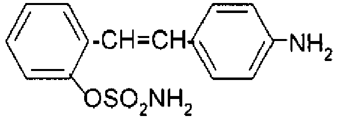

### Beispiel 1.12: (E)-4-Amino-2'-sulfamoyloxy-stilben (aus Beispiel 1.8 E)

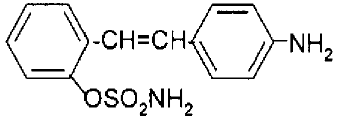

### Endprodukte

### Beispiel 1.13: (Z)-4-(3,3-Dimethyltriazenyl-1)-2'-natriumsulfooxy-stilben

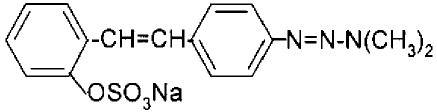

Zu einer Lösung aus 15,6 g (0,05 Mol) cis-Aminostilben aus Beispiel 1.9, 100 ml Wasser und 13 ml konz. Salzsäure tropft man bei 0°C eine Lösung aus 3,5 g Natriumnitrit und 5 ml Wasser und lässt 10 Minuten nachrühren. Dann tropft man die resultierende Diazoniumsalzlösung rasch in eine Lösung aus 30 g Natriumcarbonat, 60 ml Wasser und 7 g 40 %-iger wässriger Dimethylaminlösung. Man lässt 40 Minuten nachrühren und engt die Reaktionslösung am Rotationsverdampfer ein. Der Rückstand wird in der gerade nötigen Menge Wasser bei 50 °C gelöst und die Titelverbindung mit einer gesättigten wässrigen Kochsalzlösung ausgefällt, abgesaugt und getrocknet. Das Produkt kann aus einer Kochsalzlösung oder aus Methanol/Wasser umkristallisiert werden.

### Analog werden hergestellt:

### Beispiel 1.14: (E)- 4-(3,3-Dimethyltriazenyl-1)-2'-natriumsulfooxy-stilben

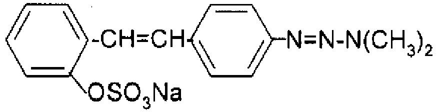

(aus Beispiel 1.10 analog Beispiel 1.13)

### Beispiel 1.15: (Z)- 4-(3,3-Dimethyltriazenyl-1)- 2'-sulfamoyloxy-stilben

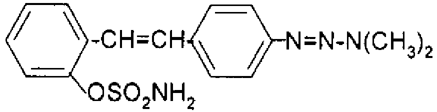

Zu einer Lösung aus 15,6 g (0,05 Mol) Z-Aminostilben aus Beispiel 1.11, 100 ml Wasser und 10 ml konz. Salzsäure tropft man bei 0°C eine Lösung aus 3,5 g Natriumnitrit und 5 ml Wasser und lässt 10 Minuten nachrühren. Dann tropft man die resultierende Diazoniumsalzlösung rasch in eine Lösung aus 30 g Natriumcarbonat, 60 ml Wasser und 7 g 40 %-iger wässriger Dimethylaminlösung. Man lässt 40 Minuten nachrühren, gibt 50 ml Wasser hinzu und extrahiert mit 300 ml Essigsäureethylester. Die organische Phase wird abgetrennt, mit 300 ml einer gesättigten wässrigen Natriumhydrogencarbonatlösung gewaschen, über Natriumsulfat getrocknet und eingeengt. Das Produkt wird chromatographisch gereinigt und anschließend ggf. aus Ethanol/Cyclohexan umkristallisiert.

### Beispiel 1.16:

### (E)- 4-(3,3-Dimethyltriazenyl-1)-2'-sulfamoyloxy-stilben (aus Beispiel 1.12)

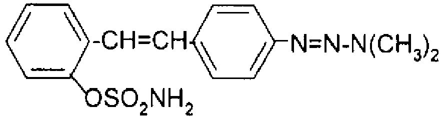

### 2. Triphenylethylen-derivate der Formel (12)

### Ausgangsprodukte

### Beispiel 2.1: [Bis(4-methoxyphenyl)]-brom-methan

(CH₃O-C₆H₄-)₂CH-Br

In eine Suspension von 100 g Bis-(4-methoxyphenyl)-carbinol und 46 g Calciumchlorid in 2 I Toluol wird bis zur Sättigung Bromwasserstoff eingeleitet. Das ausgeschiedene Salz wird abgesaugt und das Filtrat eingeengt. Der gelbe ölige Rückstand wird roh weiter verarbeitet.

### Beispiel 2.2: [Bis(4-methoxyphenyl)-methyl]-triphenylphosphoniumbromid

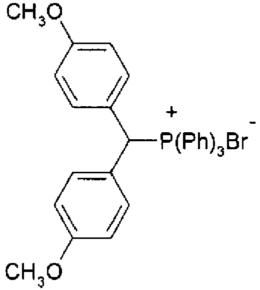

Aus dem Bromid des Beispiels 2.1 wird analog Beispiel 1.5 die Titelverbindung hergestellt.

### Beispiel 2.3: 1,1-[Bis-(4-methoxyphenyl)]-2-(4-nitrophenyl)-ethen

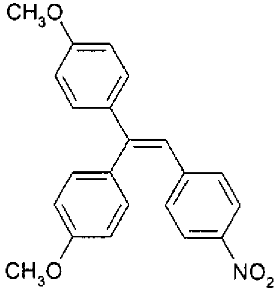

4,5 g (0,1 Mol) Natriumhydrid (53 %-ige Öldispersion) werden unter absolut trockenen Bedingungen in 50 ml DMSO eingetragen. Bis zur Beendigung der Wasserstoffentwicklung wird auf 70 bis 80°C erhitzt. Unter Eiskühlung werden 57 g (0,1 Mol) des Phosphoniumsalzes aus Beispiel 2.2, gelöst in 150 ml DMSO, zugetropft und 1 Std. nachgerührt. Anschließend werden 15,1 g (0,1 Mol) 4-Nitrobenzaldehyd, gelöst in 50 ml DMSO, zugetropft und 15 Std. bei Raumtemperatur nachgerührt. Dann gibt man 600 ml Wasser hinzu, extrahiert mit 500 ml Essigsäureethylester, entfernt das Lösungsmittel am Rotationsverdampfer und trennt das Produkt über eine Aluminiumoxidsäule vom Triphenylphosphinoxid ab. Eluiert wird mit Toluol/Essigsäureethylester (9:1). Die zweite Fraktion wird am Rotationsverdampfer vom Lösungsmittel befreit und als Rohprodukt weiter verarbeitet.

### Beispiel 2.4: 1,1-[Bis-(4-Methoxyphenyl)]-2-(4-aminophenyl)-ethen

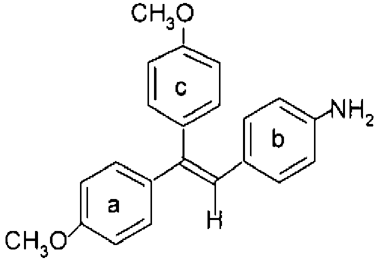

15,26 g (0,042 mol) der Nitroverbindung aus Beispiel 2.4 werden in 500 ml Ethanol zum Sieden erhitzt, mit 66 g einer 80 % Hydrazinhydratlösung (1,06 mol N₂H₄) versetzt und nach raschem Abkühlen auf 50°C so lange unter Rühren mit frisch bereitetem und in neutralem in Ethanol suspendiertem Raney-Nickel versetzt, bis bei erneuter Katalysatorzugabe keine Gasentwicklung mehr beobachtet wird. Anschließend erhitzt man noch eine Std. unter Rückfluss und filtriert das heiße Reaktionsgemisch ab. Nach Waschen des Filterrückstandes mit 250 ml heißem Ethanol werden die vereinigten Filtrate im Vakuum bis zur Trockne eingeengt und der Rückstand ggf. aus Ethanol umkristallisiert

### Beispiel 2.5: 1,1-[Bis-(4-hydroxyphenyl)]-2-(4-aminophenyl)-ethen

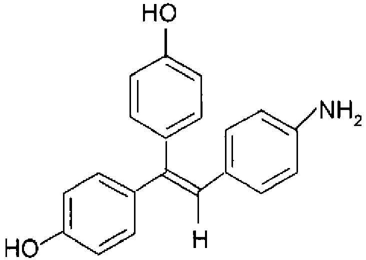

Zu einer Mischung aus 33 g (0,1 Mol) der Verbindung des Beispiels 2.4 und 66 ml Pyridin werden 84 g konz. Salzsäure getropft. Dann wird die Reaktionslösung 3 Stunden auf 150°C erhitzt und anschließend am Rotationsverdampfer bis zur Trockne eingeengt, mit 200 ml Wasser aufgenommen und neutralisiert. Der feste Reaktionsprodukt wird abgesaugt und getrocknet. Das Rohprodukt wird weiter umgesetzt.

### Beispiel 2.6:

### 1,1-[Bis-(4-hydroxyphenyl)]-2-[4-(3,3-dimethyltriazyl-1)-phenyl]-ethen

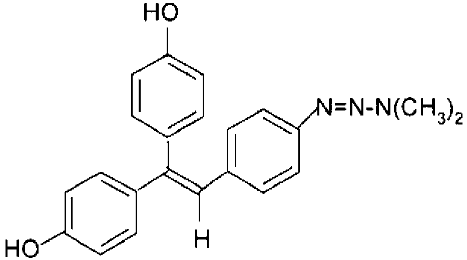

13,55 g (0,05 Mol) 1,1-[Bis-(4-hydroxyphenyl)]-2-(4-aminophenyl)-ethen aus Beispiel 2.5 werden in einer Lösung aus 13 ml konzentrierter Salzsäure und 80 ml Wasser gelöst. Dazu wird unter Rühren bei 0 bis 5°C langsam eine Lösung aus 10 ml Wasser und 3,5 g Natriumnitrit getropft. Dann tropft man die resultierende Diazoniumsalzlösung bei 0 bis 5°C rasch in eine Lösung aus 15 g Natriumcarbonat, 60 ml Wasser und 7 g 40 %-iger wässriger Dimethylaminlösung. Anschließend wird 30 Minuten nachgerührt, neutralisiert und die Titelsubstanz als Feststoff abgesaugt, ggf. aus Nitropropan umkristallisiert und getrocknet. Das Rohprodukt wird weiterverarbeitet.

### Endprodukte

### Beispiel 2.7: 1,1-[Bis-4-(natriumsulfooxy-phenyl)]-2-[4-(3,3-dimethyltriazenyl-1)-phenyl]-ethen

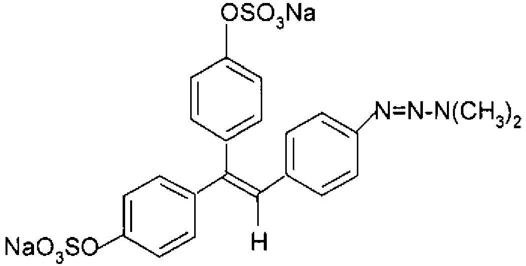

3,60 g (0,01 Mol) der Verbindung aus Beispiel 2.6 und 6,6 g (0,03 Mol) Natriumdisulfat werden mit 10 ml Dimethylformamid und 13 ml Tetrahydrofuran versetzt und 30 Stunden lang bei Raumtemperatur gerührt. Dann setzt man der Reaktionsmischung 20 ml einer 1 n wässrigen Natriumcarbonatlösung zu, verrührt die Mischung, saugt ab und engt das Filtrat unter vermindertem Druck ein. Der Rückstand wird in der gerade nötigen Menge Wasser gelöst, über Aktivkohle filtriert, das Filtrat am Rotationsverdampfer eingeengt und der Rückstand aus Methanol/Wasser umkristallisiert.

### Beispiel 2.8:

### 1,1-[Bis-4-(sulfamoyloxy-phenyl)]-2-[4-(3,3-dimethyltriazenyl-1)-phenyl]-ethen

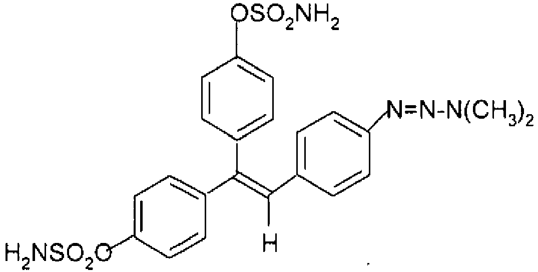

Zu einer Lösung von 3,60 g (0,01 Mol) der Verbindung aus Beispiel 2.6 in 20 ml wasserfreiem Dimethylacetamid werden bei 0 bis 5°C 4 g (0,033 Mol) Sulfamoylchlorid getropft. Das Reaktionsgemisch wird 3 Stunden bei Raumtemperatur gerührt. Dann wird mit 100 ml Essigsäureethylester extrahiert, die organische Phase mit einer gesättigten wässrigen Natriumhydrogencarbonatlösung gewaschen, über Natriumsulfat getrocknet und das Lösungsmittel am Rotationsverdampfer abdestiliert. Der Rückstand wird mit einem Essigsäureethylester/Petrolether-Gemisch verrieben, zur Kristallisation gebracht und abgesaugt.

### 3. Flavonoidderivate der Formel (14) (nicht gemäß der Erfindung)

### Ausgangsprodukte

### Beispiel 3.1: 7,4'-Di-hydroxy-3'-nitro-isoflavon

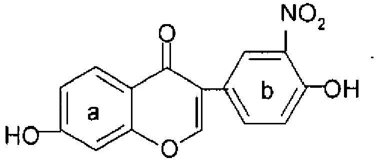

20 ml einer Mischung von konzentrierter Schwefelsäure und Salpetersäure (3:1) werden tropfenweise zu einer Lösung von 1 g Daidzein in 200 ml trockenem Ethanol unter kräftigem Rühren bei Raumtemperatur zugegeben. Es wird 2 Std. nachgerührt, dann werden 400 ml Wasser zugesetzt. Die Verbindung scheidet sich ab, wird abgesaugt und mit Wasser neutral gewaschen. Das gelbe Produkt wird aus Alkohol umkristallisiert.

Die 2'-Nitroisoflavone werden nach S. Tappmeyer elektronische Dissertation erhalten: http://www.diss.fu-berlin.de/2004/287/index.html hergestellt.

### Synthese der Sulfate

Die folgenden Beispiele werden analog den Beispielen 1.1 durchgeführt. Die Titelverbindungen werden als Rohprodukte weiter umgesetzt.

### Beispiel 3.2: 7-Natriumsulfooxy-4'-methoxy-2'-nitro-isoflavon

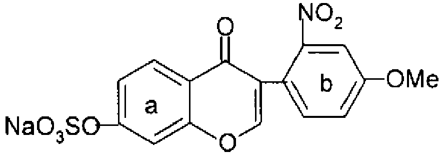

### Beispiel 3.3: 7,4'-Di-natriumsulfooxy-2'-nitro-isoflavon

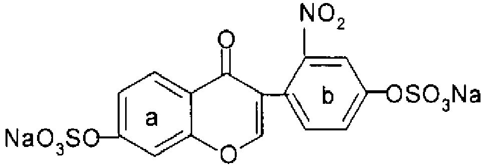

### Beispiel 3.4: 7,4'-Di-natriumsulfooxy-3'-nitro-isoflavon

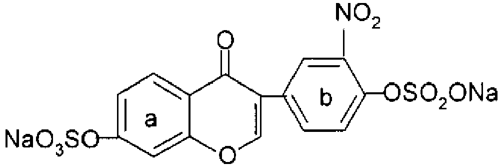

### Beispiel 3.5: 7-Methoxy-3'-nitro-4'-natriumsulfooxy-isoflavon

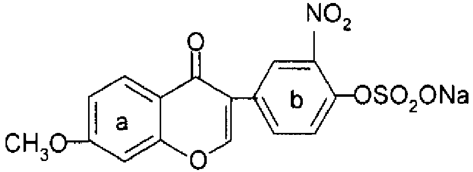

### Beispiel 3.6: 7-Natriumsulfooxy-3'-nitro-4'-methoxy-isoflavon

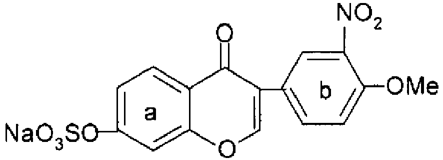

### Synthese der Oxysulfamate

Die folgenden Beispiele werden analog den Beispielen 1.3 durchgeführt. Die Titelverbundungen werden als Rohprodukte weiter umgesetzt.

### Beispiel 3.7: 7, 4'-Disulfamoyloxy-2'-nitro-isoflavon

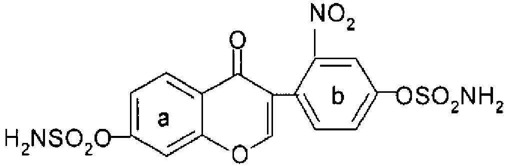

### Beispiel 3.8: 7, 4'-Disulfamoyloxy-3'-nitro-isoflavon

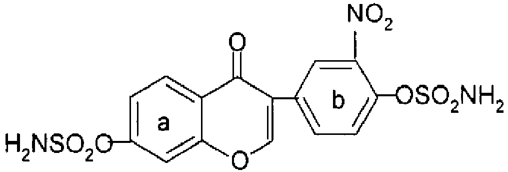

### Reduktion der Nitro-isoflavone zu den Amino-isoflavonen

Die Reduktion der Nitroverbindungen erfolgt durch katalytische Reduktion mit Wasserstoff/Pd/C in Ethanol.

### Beispiel 3.9: 7-Natriumsulfooxy-2'-amino-4'-methoxy-isoflavon

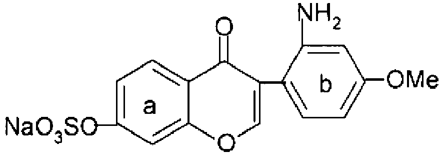

### Analog werden hergestellt:

### Beispiel 3.10 7, 4'-Di-natriumsulfooxy-2'-amino-isoflavon

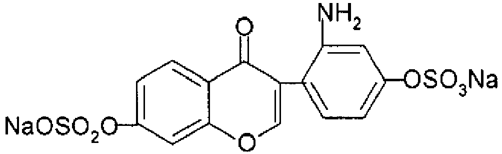

### 3.11 7,4'-Di-natriumsulfooxy-3'-amino-isoflavon

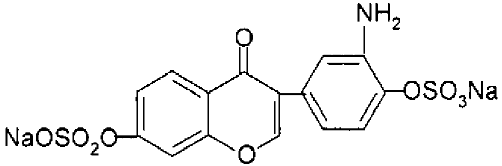

### 3.12 7-Methoxy-3'-amino-4'natriumsulfooxy-isoflavon

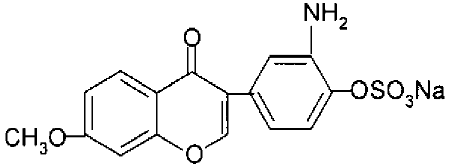

### 3.13 7-Natriumsulfooxy-3'-amino-4'-methoxy-isoflavon

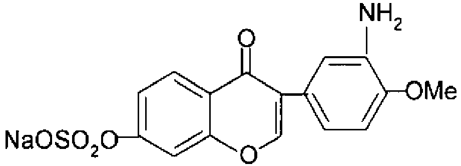

### 3.14 7-Natriumsulfooxy-2'-amino-4'methoxy-isoflavon

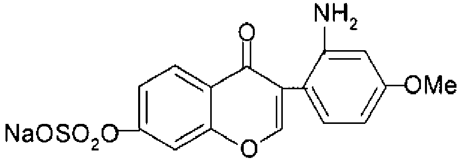

### 3.15 7,4'- Di-sulfamoyloxy-3'-amino-isoflavon

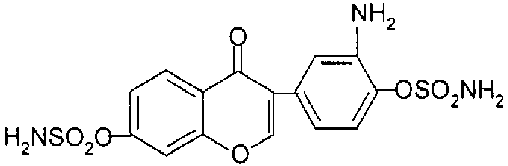

### 3.16 7,4'-Di-sulfamoyloxy-2'-amino-isoflavon

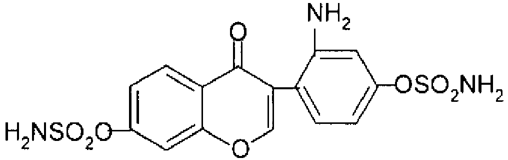

### 3.17 7-Sulfamoyloxy-3'-amino-4'-methoxy-isoflavon

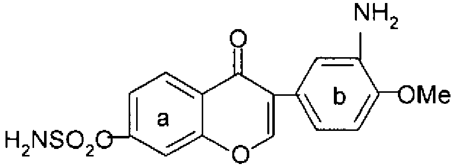

Endprodukte

### Beispiel 3.18: 7-Natriumsulfooxy-3'-[(3,3-dimethyltriazenyl-1)-4'-methoxy)]-isoflavon

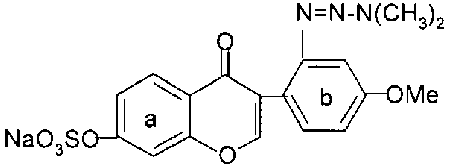

Zu einer Lösung aus 4 g (0,02 Mol) des Amino-isoflavons aus Beispiel 3.14, 30 ml Wasser und 5 ml konz. Salzsäure tropft man bei 0°C eine Lösung aus 2 g Natriumnitrit und 20 ml Wasser, lässt 10 Minuten nachrühren und tropft die resultierende Diazoniumsalzlösung rasch in eine Lösung aus 8 g Natriumcarbonat, 15 ml Wasser und 2 g einer 40 %-iger wässriger Dimethylaminlösung. Man lässt 60 Minuten nachrühren, saugt das kristalline Reaktionsprodukt ab und kristallisiert aus wenig Wasser um.

Die folgenden erfindungsgemäßen Triazeno-isoflavone werden analog Beispiel 3.18 aus den oben genannten Amino-isoflavonen hergestellt.

### Beispiel 3.19: 7,4'-Di-natriumsulfooxy-2'-(3,3-dimethyltriazenyl-1)-isoflavon

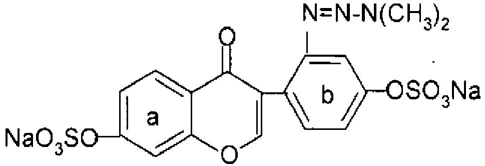

### Beispiel 3.20: 7,4'-Di-natriumsulfooxy-3'-(3,3-dimethyltriazenyl-1)-isoflavon

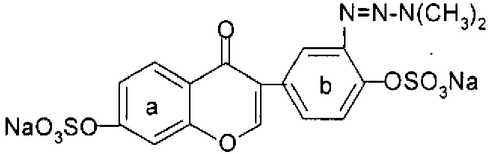

### Beispiel 3.21: 7-Methoxy-3'-(3,3-dimethyltriazenyl-1)-4'-natriumsulfooxy-isoflavon

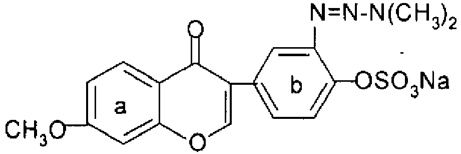

### Beispiel 3.22: 7-Natriumsulfooxy 3'-(3,3-dimethyltriazenyl-1)-4'-methoxy-isoflavon

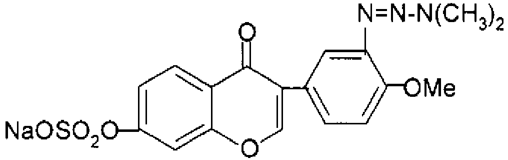

### Beispiel 3.23: 7,4'-Sulfamoyloxy- 3'-(3,3-dimethyltriazenyl-1)- isoflavon

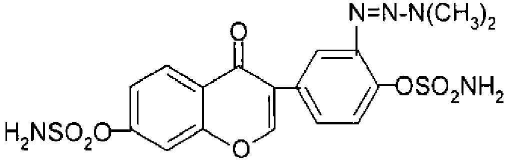

### Beispiel 3.24: 7-Sulfamoyloxy-2'-(3,3-dimethyltriazenyl-1)-4'-methoxy-isoflavon

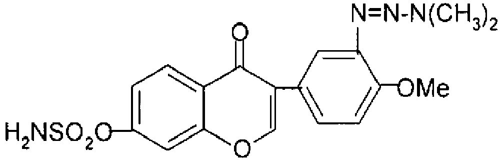

### 4. Unverbrückte oder mit Atomen oder zweiatomigen Gruppen verbrückte Diphenylderivate (18):

### Ausgangsprodukte:

### Beispiel 4.1: 3-Methyl-4-nitro-4'-(3-methyl-4-nitro-benzoyloxy)-benzophenon

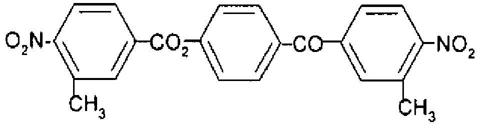

Eine Mischung aus 122 g Phenetol (1 Mol), 400 g 3-Methyl-4-nitro-benzoylchlorid (2 Mol) und 1500 ml 1,2-Dibromethan wird vorgelegt.

Dazu werden unter Kühlung bei 15-20°C in Portionen 400 g Aluminiumchlorid eingetragen (exotherm!). Anschließend wird die Reaktionsmischung stufenweise je 2 Stunden bei: 20°-30°C, 40°C und 80°C gerührt.

Aufarbeitung: Reaktionsgemisch auf O°C kühlen und langsam in 1,6 kg Eis eintragen. Dann dem Reaktionsgemisch 100 ml konz. Salzsäure zusetzen, umrühren und stehen lassen. Die überstehende Lösung abdekantieren und das Festprodukt absaugen und mit Wasser waschen.

Den Filterrückstand mit 400 ml Aceton aufschlemmen, auf O°C kühlen, absaugen und trocknen.

Reinigung: Aus Nitropropan umkristallisieren:

### Beispiel 4.2: 4-Hydroxy-3'-methyl-4'-nitro-benzophenon

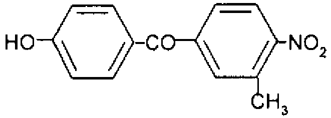

322 g (1 Mol) 4-Nitro-4'-(4-nitrobenzoyloxy)-benzophenon (Beispiel 4.1) werden in 1,3 l Wasser, 200ml Ethanol und 85 g Natriumhydroxyd so lange erhitzt, bis sich der Feststoff aufgelöst hat.

Aufarbeitung: Die Reaktionsmischung wird über Activkohle/Kieselgur heiß abgesaugt. Aus dem Filtrat wird durch Zugabe von 120 ml konzentrierter Salzsäure onko 0640 zusammen mit 4-Nitrobenzoesäure ausgefällt. Der Feststoff wird abgesaugt und mit Wasser gewaschen. Der Filterrückstand wird mit 140 g Natriumhydrogenkarbonat und 900 ml Wasser 1 Std. bei 70 gerührt, heiß abgesaugt, mit 1l heißem Wasser gewaschen und getrocknet.

### Beispiel 4.3: 4-Amino-3-methyl-4'-hydroxy-benzophenon

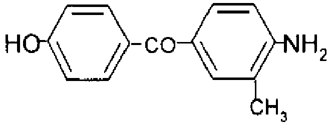

25,8 g (0,1 Mol) 4-Hydroxy-3'-methyl-4'-nitro-benzophenon (Beispiel 4.2) werden in 200 ml Ethanol gelöst. Bei 80°C wird unter Rühren eine Lösung von 35 g Natriumsulfid in 40 ml Wasser langsam zugetropft. Anschließend wird die Reaktionslösung 2 Std. am Rückfluss erhitzt.

Aufarbeitung: Die Reaktionslösung wird auf 1 l Wasser gegossen, auf 20° C gekühlt, und das ausgefallene Produkt abgesaugt, getrocknet und aus Toluol umkristallisiert.

### Beispiel 4.4: 4- Hydroxy-4'-(3,3-dimethyltriazenyl-1)-benzophenon

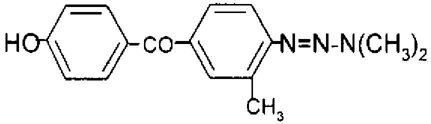

57 g (0,25 Mol) 4-Amino-3-methyl 4'-hydroxy-benzophenon werden in einer Lösung aus 75 ml konzentrierter Salzsäure und 400 ml Wasser gelöst. Dann wird eine Lösung aus 25 ml Wasser und 17,25 g Natriumnitrit bei 0 bis 5°C langsam unter Rühren hinzugetropft. Diese Diazoniumsalzlösung wird dann bei 0 bis 5°C unter Rühren zu einer Lösung getropft, die man aus 75 g Natriumcarbonat, 150 ml Wasser und 35 g einer 40 %-igen wässrigen Dimethylaminlösung hergestellt hat. Man rührt 1 Stunde nach neutralisiert und saugt den ausgefallenen Feststoff ab, trocknet und kristallisiert ihn aus n-Hexan/Toluol um.

### Endprodukte

### Beispiel 4.5: 4-(3,3-Dimethtriazenyl-1)-4'-natriumsulfooxy-diphenylsulfon (nicht gemäß der Erfindung)

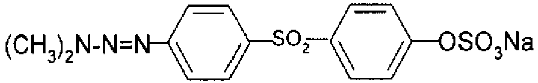

4 g (0,01 Mol) 4-Hydroxy-4'-(3,3-dimethyl-triazenyl-1)-diphenylsulfon und 3,3 g (0,15 Mol) Natriumdisulfat werden mit 20 ml Dimethylformamid und 25 ml Tetrahydrofuran versetzt und 30 Stunden lang bei Raumtemperatur gerührt. Dann setzt man der Reaktionsmischung 20 ml einer 1 n wässrigen Natriumcarbonatlösung zu, verrührt die Mischung, saugt ab und engt das Filtrat unter vermindertem Druck ein. Der Rückstand wird in der gerade nötigen Menge Wasser gelöst, über Aktivkohle filtriert, das Filtrat am Rotationsverdampfer eingeengt und der Rückstand aus Methanol/Wasser oder einer Natriumchloridlösung umkristallisiert.

Analog können hergestellt werden:

### Beispiel 4.6: 4-(3,3-Dimethyltriazenyl-1)-2'-natriumsulfooxy-benzophenon

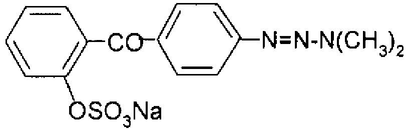

### Beispiel 4.7:

### 2,6-Dibrom-4-natriumsulfooxy-4'-(3,3-dimethyltriazenyl-1)- benzophenon

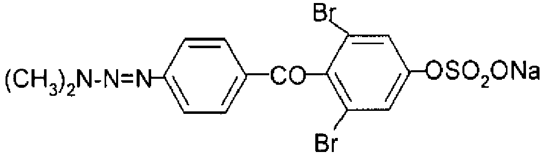

### Beispiel 4.8:

### 2,6-Dimethyl-4-natriumsulfooxy-4'-(3,3-dimethyltriazenyl-1)-benzophenon

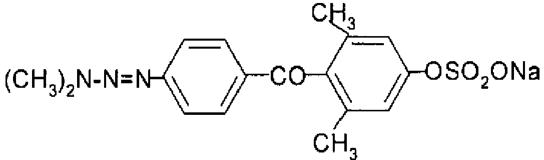

### Beispiel 4.9: 4-(3,3-Dimethyltriazenyl-1)-4'-natriumsulfooxy-biphenyl (nicht gemäß der Erfindung)

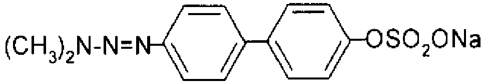

### Beispiel 4.10: 4-(3,3-Dimethyltriazenyl-1)-4'-natriumsulfooxy-diphenylsulfid (nicht gemäß der Erfindung)

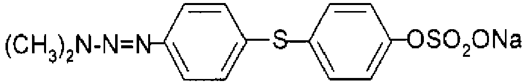

### Beispiel 4.11: 4-(3,3-Dimethyltriazenyl-1)-4'-natriumsulfooxy-diphenylsulfoxid (nicht gemäß der Erfindung)

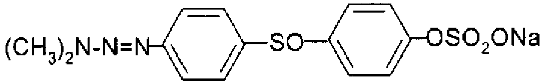

### Beispiel 4.12: 4-(3,3-Dimethyltriazenyl-1)-4'-natriumsulfooxy-diphenylsulfon (nicht gemäß der Erfindung)

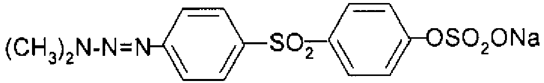

### Beispiel 4.13: 4-(3,3-Dimethyltriazenyl-1)-4'-sulfamoyloxy-benzophen

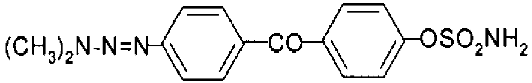

Zu einer Lösung von 13,4 g (005 Mol) 4-(3,3-Dimethyitriazenyl-1)-4'-hydroxy-benzophenon (Beispiel 4.6) in 100 ml Dimethylacetamid werden 8,6 g (0,075 Mol) Chlorsulfonsäureamid bei 0°C getropft. Man rührt 1 Std. bei 0°C und anschließend 12 Std. bei Raumtemperatur nach. Dann gibt man eine gesättigte wässrige Ammoniumchloridlösung hinzu, extrahiert mit Essigsäureethylester, trocknet mit Natriumsulfat, filtriert und zieht das Lösungsmittel ab. Das Produkt wird aus Cyclohexan/ Essigsäureethylester umkristallisiert. Analog können hergestellt werden:

### Beispiel 4.14: 4-(3,3-Dimethyltriazenyl-1)-2'-sulfamoyloxy-benzophenon

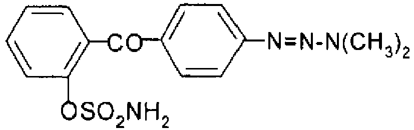

### Beispiel 4.15: 4-(3,3-Dimethyltriazenyl-1)-4'-sulfamoyloxy-diphenylsulfoxid (nicht gemäß der Erfindung

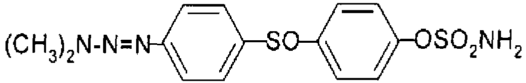

### Beispiel 4.16: 4-(3,3-Dimethyltriazenyl-1)-4'-sulfamoyloxy-diphenylsulfon (nicht gemäß der Erfindung)

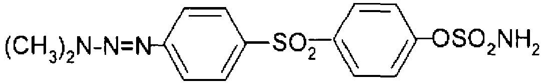

## Patentansprüche

1. Diphenylderivate der Formel 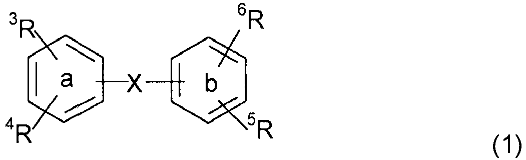 worin
X CO oder -CR⁷=CR⁸-,
R³, R⁶ unabhängig voneinander Wasserstoff, Hydroxyl-, C₁-C₄-Alkyl, C₁-C₄-Alkoxy, C₁-C₄-Alkyl-S-, C₁-C₄-Alkyl-SO-, C₁-C₄-Alkyl-SO₂-, Halogen, Nitro, Cyano, oder eine -OSO₂Y-Gruppe,
R⁴, R⁵ unabhängig voneinander eine -N=N-N(R²)₂- oder eine -OSO₂Y-Gruppe,
R⁷ Wasserstoff, Methyl, Ethyl oder einen durch die Reste R⁹, R¹⁰ substituierten Phenylrest, der direkt an das R⁷-tragende C-Atom geknüpft ist,
R⁸ Wasserstoff, Ethyl, CN, NO₂, -CH₂CH₂-Halogen oder CH₂CH₂OH,
R⁹, R¹⁰ unabhängig voneinander Wasserstoff, Hydroxyl-, C₁-C₄-Alkyl, C₁-C₄-Alkoxy, Halogen, Nitro, Cyano, eine -N=N-N(R²)₂- oder eine -OSO₂Y-Gruppe,
Y OH oder N(R¹)₂,
R¹ Wasserstoff, Methyl oder Ethyl und
R² Methyl oder Ethyl
bedeuten,
mit der Maßgabe, dass pro Gesamtmolekül (1) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

2. Diphenylderivate nach Anspruch 1 der Formel 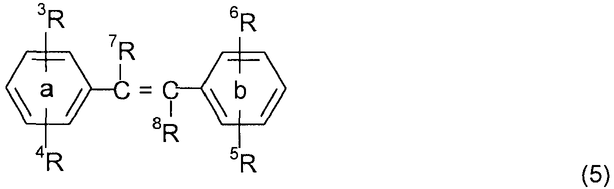 worin
R³, R⁶ unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, Halogen, Nitro, Cyano oder eine -OSO₂Y-Gruppe,
R⁴, R⁵ unabhängig voneinander eine -N=N-N(R²)₂- oder eine -OSO₂Y-Gruppe,
R⁷ Wasserstoff, Methyl, Ethyl oder einen durch die Reste R⁹, R¹⁰ substituierten Phenylrest, der direkt an das R⁷-tragende C-Atom geknüpft ist,
R⁸ Wasserstoff, Ethyl, CN, NO₂, -CH₂CH₂-Halogen oder CH₂CH₂OH,
R⁹, R¹⁰ unabhängig voneinander Wasserstoff, Hydroxyl, C₁-C₄-Alkyl, C₁-C₄-Alkoxy, Halogen, Nitro, Cyano, eine -N=N-N(R²)₂- oder eine -OSO₂Y-Gruppe,
Y OH oder N(R¹)₂,
R¹ Wasserstoff, Methyl oder Ethyl und
R² Methyl oder Ethyl
bedeuten,
mit der Maßgabe, dass pro Gesamtmolekül (5) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

3. Diphenylderivate Anspruch 1 der Formel 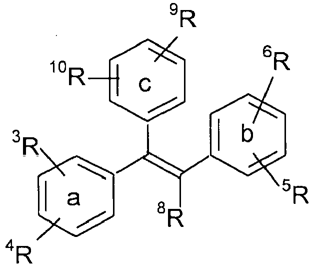 (10) worin
R³, R⁶ unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, Halogen, Nitro, Cyano oder eine -OSO₂Y-Gruppe,
R⁴, R⁵ unabhängig voneinander eine -N=N-N(R²)₂- oder eine -OSO₂Y-Gruppe,
R⁸ Wasserstoff, Ethyl, CN, NO₂, -CH₂CH₂-Halogen oder CH₂CH₂OH,
R⁹, R¹⁰ unabhängig voneinander Wasserstoff, Hydroxyl, C₁-C₄-Alkyl, C₁-C₄-Alkoxy, Halogen, Nitro, Cyano, eine -N=N-N(R²)₂- oder eine -OSO₂Y-Gruppe,
Y OH oder N(R¹)₂,
R¹ Wasserstoff, Methyl oder Ethyl und
R² Methyl oder Ethyl bedeuten,
mit der Maßgabe, dass pro Gesamtmolekül (10) ein bis zwei -N=N-N(R²)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

4. Diphenylderivate nach Anspruch 1 der Formel 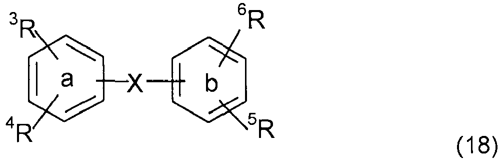 worin
X CO,
R³, R⁶ unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, Halogen, Nitro, Cyano oder eine -OSO₂Y-Gruppe,
R⁴, R⁵ unabhängig voneinander eine -N=N-N(R²)₂- oder eine -OSO₂Y-Gruppe,
Y OH oder N(R¹)₂,
R¹ Wasserstoff, Methyl oder Ethyl und
R² Methyl oder Ethyl
bedeuten,
mit der Maßgabe, dass pro Gesamtmolekül (18) ein bis zwei -N=N-N(²R)₂- und ein bis zwei -OSO₂Y-Gruppen an beliebigen Ring-Kohlenstoffatomen von aromatischen Ringen stehen,
sowie deren Salze, freie Säuren, Solvate und die Solvate dieser Salze und die Solvate dieser freien Säuren.

5. Diphenylderivat nach Anspruch 1 oder 4, wobei das Diphenylderivat 4-(3,3-dimethyl-triazenyl-1)-4'-natriumsulfooxy-benzopheneon gemäß der nachfolgenden Formel 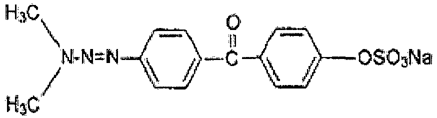 ist.

6. Verfahren zur Herstellung von Diphenylderivaten, die pro Molekül (i) mindestens eine Dialkyltriazenyl- und (ii) mindestens eine Sulfooxy- und/oder mindestens eine Sulfamoyloxygruppe enthalten, und deren Salze, Solvate und Solvate dieser Salze, **dadurch gekennzeichnet, dass** Aminophenylderivate der Formel 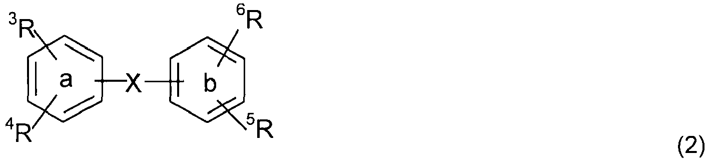 worin
X CO oder -CR⁷=CR⁸-,
R³, R⁶ unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂-, Halogen Nitro, Cyano oder eine -OSO₂Y-Gruppe,
R⁴, R⁵ unabhängig voneinander eine -NH₂- oder eine -OSO₂Y-Gruppe,
R⁷ Wasserstoff, Methyl, Ethyl oder einen durch die Reste R⁹, R¹⁰ substituierten Phenylrest, der direkt an das R⁷-tragende C-Atom geknüpft ist,
R⁸ Wasserstoff, Ethyl, CN, NO₂, -CH₂CH₂-Halogen oder CH₂CH₂OH,
R⁹, R¹⁰ unabhängig voneinander Wasserstoff, Hydroxyl, C₁-C₄-Alkyl, C₁-C₄-Alkoxy, Halogen, Nitro, Cyano, eine -NH₂- oder eine -OSO₂Y-Gruppe,
Y OH oder N(R¹)₂ und
R¹ Wasserstoff, Methyl oder Ethyl
bedeuten,
mit der Maßgabe, dass pro Gesamtmolekül (2) ein bis zwei NH₂- und ein bis zwei - OSO₂Y-Gruppen an beliebigen Ring-Kohlenstoffatomen der aromatischen Ringen stehen,
in Gegenwart starker Säure bei niedriger Temperatur in wässrig-saurer Lösung mit einem Diazotierungsmittel zu Diazoniumsalzen der Formel 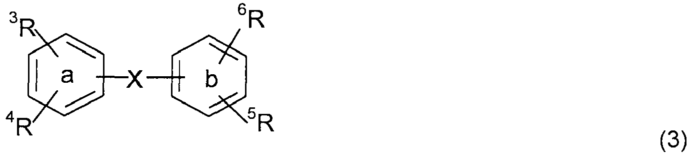 worin
X CO oder -CR⁷=CR⁸-,
R³, R⁶ unabhängig voneinander Wasserstoff, Hydroxyl, C₁-C₄-Alkyl, C₁-C₄-Alkoxy, C₁-C₄-Alkyl-S-, C₁-C₄-Alkyl-SO-, C₁-C₄-Alkyl-SO₂, Halogen, Nitro, Cyano oder eine -OSO₂Y-Gruppe,
R⁴, R⁵ unabhängig voneinander eine -N₂⁺An⁻- oder eine -OSO₂Y-Gruppe,
R⁷ Wasserstoff, Methyl, Ethyl oder einen durch die Reste R⁹, R¹⁰ substituierten Phenylrest, der direkt an das R⁷-tragende C-Atom geknüpft ist,
R⁸ Wasserstoff, Ethyl, CN, NO₂, -CH₂CH₂-Halogen oder CH₂CH₂OH,
R⁹, R¹⁰ unabhängig voneinander Wasserstoff, Hydroxyl, C₁-C₄-Alkyl, C₁-C₄-Alkoxy, Halogen, Nitro, Cyano, eine -N₂⁺An⁻- oder eine -OSO₂Y-Gruppe,
An⁻ ein Halogen- oder ½ Sulfat (SO₄²⁻)-Anion,
Y OH oder N(¹R)₂ und
R¹ Wasserstoff, Methyl oder Ethyl
bedeuten,
mit der Maßgabe, dass pro Gesamtmolekül (3) ein bis zwei Diazonium- und ein bis zwei -OSO₂Y-Gruppen an beliebigen Ring-Kohlenstoffatomen von aromatischen Ringen stehen, diazotiert, und dann die erhaltenen Diazoniumsalze (3) mit Dialkylaminen der Formel
HN(R²)₂ (4)
worin R² für Methyl oder Ethyl steht, in Gegenwart von Säurebindemittel umgesetzt werden, gegebenenfalls gefolgt durch Herstellung der Salze aus den erhaltenen Verbindungen und gegebenenfalls gefolgt durch Freisetzung der Hydroxyl- und/oder Säuregruppen aus diesen Salzen.

7. Diphenylderivate nach einem der Ansprüche 1 bis 5 zur Bekämpfung von Tumoren der Haut und der von Sexualhormonen abhängigen Zielorgane bei Menschen und Tieren.

8. Arzneimittel, enthaltend mindestens ein Diphenylderivat nach einem der Ansprüche 1 bis 5 und dessen Salze, Solvate und Solvate dieser Salze gegebenenfalls zusammen mit einem oder mehreren pharmakologisch unbedenklichen Hilfs- oder Trägerstoffen.

## Claims

1. Diphenyl derivatives of formula 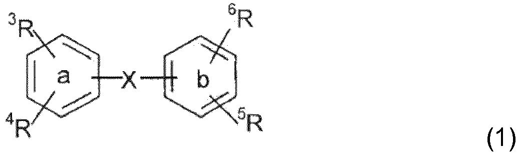 wherein
X CO or -CR⁷=CR⁸-,
R³, R⁶ independently of one another are hydrogen, hydroxy-, C₁-C₄-alkyl, C₁-C₄-alkoxy, C₁-C₄-alkyl-S-, C₁-C₄-alkyl-SO-, C₁-C₄-alkyl-SO₂-, halogen, nitro, cyano, or an -OSO₂Y-group,
R⁴, R⁵ independently of one another are a -N=N-N(R²)₂- or an -OSO₂Y-group,
R⁷ is hydrogen, methyl, ethyl or a phenyl residue substituted with the residues R⁹, R¹⁰, which is directly linked to the C atom bearing the R⁷,
R⁸ is hydrogen, ethyl, CN, NO₂, -CH₂CH₂-halogen or CH₂CH₂OH,
R⁹, R¹⁰ independently of one another are hydrogen, hydroxy-, C₁-C₄-alkyl, C₁-C₄-alkoxy, halogen, nitro, cyano, a -N=N-N(R²)₂- or an -OSO₂Y-group,
Y is OH or N(R¹)₂,
R¹ is hydrogen, methyl or ethyl and
R² is methyl or ethyl,
with the proviso that for each total molecule (1), one to two -N=N-N(R²)₂- groups and one to two -OSO₂Y- groups are located on any arbitrary ring carbon atoms of aromatic rings,
as well as their salts, free acids, solvates and the solvates of these salts and the solvates of these free acids.

2. Diphenyl derivatives according to claim 1 of formula 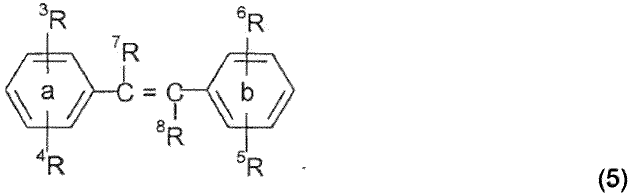 wherein
R³, R⁶ independently of one another are hydrogen, hydroxy, C₁-C₄-alkyl, C₁-C₄-alkoxy, C₁-C₄-alkyl-S-, C₁-C₄-alkyl-SO-, C₁-C₄-alkyl-SO₂-, halogen, nitro, cyano or an -OSO₂Y-group,
R⁴, R⁵ independently of one another are a -N=N-N(R²)₂- or an -OSO₂Y-group,
R⁷ is hydrogen, methyl, ethyl or a phenyl residue substituted with the residues R⁹, R¹⁰, which is directly linked to the C atom bearing the R⁷,
R⁸ is hydrogen, ethyl, CN, NO₂, -CH₂CH₂-halogen or CH₂CH₂OH,
R⁹, R¹⁰ independently of one another are hydrogen, hydroxy, C₁-C₄-alkyl, C₁-C₄-alkoxy, halogen, nitro, cyano, a -N=N-N(R²)₂- or an -OSO₂Y-group,
Y is OH or N(R¹)₂,
R¹ is hydrogen, methyl or ethyl and
R² is methyl or ethyl,
with the proviso that for each total molecule (5) one to two -N=N-N(R²)₂- and one to two -OSO₂Y- groups are located on any arbitrary ring carbon atoms of aromatic rings,
as well as their salts, free acids, solvates and the solvates of these salts and the solvates of these free acids.

3. Diphenyl derivatives according to claim 1 of formula 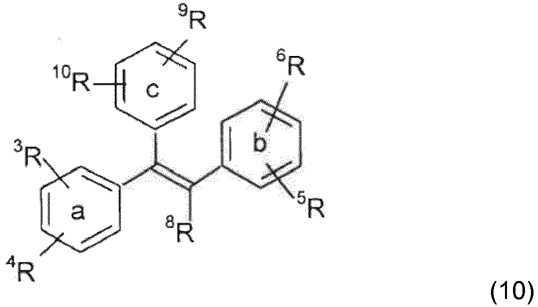 wherein
R³, R⁶ independently of one another are hydrogen, hydroxy, C₁-C₄-alkyl, C₁-C₄-alkoxy, C₁-C₄-alkyl-S-, C₁-C₄-alkyl-SO-, C₁-C₄-alkyl-SO₂-, halogen, nitro, cyano or an -OSO₂Y-group,
R⁴, R⁵ independently of one another are a -N=N-N(R²)₂- or an -OSO₂Y-group,
R⁸ is hydrogen, ethyl, CN, NO₂, -CH₂CH₂-halogen or CH₂CH₂OH,
R⁹, R¹⁰ independently of one another are hydrogen, hydroxy-, C₁-C₄-alkyl, C₁-C₄-alkoxy, halogen, nitro, cyano, a -N=N-N(R²)₂- or an -OSO₂Y-group,
Y is OH or N(R¹)₂,
R¹ is hydrogen, methyl or ethyl and
R² is methyl or ethyl,
with the proviso that for each total molecule (10) one to two -N=N-N(R²)₂- and one to two -OSO₂Y-groups are located on any arbitrary ring carbon atoms of aromatic rings, as well as their salts, free acids, solvates and the solvates of these salts and the solvates of these free acids.

4. Diphenyl derivatives according to claim 1 of formula 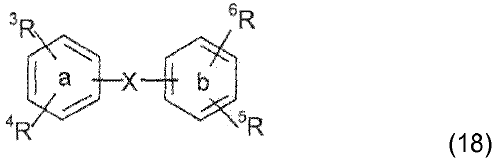 wherein
X CO,
R³, R⁶ independently of one another are hydrogen, hydroxy, C₁-C₄-alkyl, C₁-C₄-alkoxy, C₁-C₄-alkyl-S-, C₁-C₄-alkyl-SO-, C₁-C₄-alkyl-SO₂-, halogen, nitro, cyano or an -OSO₂Y-group,
R⁴, R⁵ independently of one another are a -N=N-N(R²)₂- or an -OSO₂Y-group,
Y is OH or N(R¹)₂,
R¹ is hydrogen, methyl or ethyl and
R² is methyl or ethyl,
with the proviso that for each total molecule (18) one to two -N=N-N(R²)₂- and one to two -OSO₂Y-groups are located on any arbitrary ring carbon atoms of aromatic rings,
as well as their salts, free acids, solvates and the solvates of these salts and the solvates of these free acids.

5. Diphenyl derivative according to claim 1 or 4, wherein the diphenyl derivative is 4-(3,3-dimethyl-triazenyl-1)-4'-sodium sulfooxy-benzophenone according to the following formula

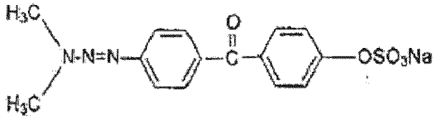

6. A method for producing diphenyl derivatives which contain per molecule (i) at least one dialkyltriazenyl- and (ii) at least one sulfamoyloxy group, and the salts, solvates and solvates of these salts, **characterized in that** aminophenyl derivatives of formula 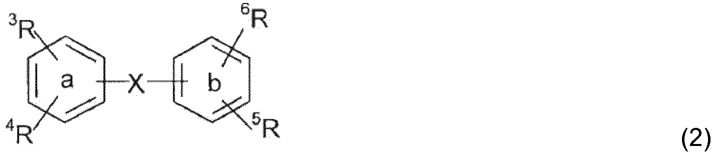 wherein
X CO or -CR⁷=CR⁸-,
R³, R⁶ independently of one another are hydrogen, hydroxy, C₁-C₄-alkyl, C₁-C₄-alkoxy, C₁-C₄-alkyl-S-, C₁-C₄-alkyl-SO-, C₁-C₄-alkyl-SO₂-, halogen, nitro, cyano or an -OSO₂Y-group,
R⁴, R⁵ independently of one another are a -NH₂- or an -OSO₂Y-group,
R⁷ is hydrogen, methyl, ethyl or a phenyl residue substituted with the residues R⁹, R¹⁰, which is directly linked to the C atom bearing the R⁷,
R⁸ is hydrogen, ethyl, CN, NO₂, -CH₂CH₂-halogen or CH₂CH₂OH,
R⁹, R¹⁰ independently of one another are hydrogen, hydroxy-, C₁-C₄-alkyl, C₁-C₄-alkoxy, halogen, nitro, cyano, a -NH₂- or an -OSO₂Y-group,
Y is OH or N(R¹)₂ and
R¹ is hydrogen, methyl or ethyl,
with the proviso that for each total molecule (2) one to two -NH₂- and one to two -OSO₂Y- groups are located on any arbitrary ring carbon atoms of aromatic rings, in the presence of strong acid at low temperature in aqueous-acidic solution are diazotized with a diazotizing agent to form diazonium salts of formula 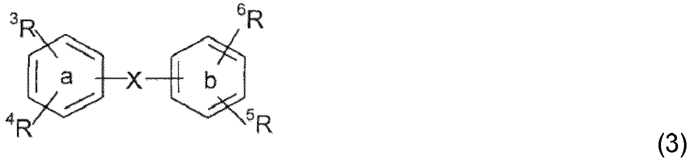 wherein
X CO or -CR⁷=CR⁸-,
R³, R⁶ independently of one another are hydrogen, hydroxy, C₁-C₄-alkyl, C₁-C₄-alkoxy, C₁-C₄-alkyl-S-, C₁-C₄-alkyl-SO-, C₁-C₄-alkyl-SO₂-, halogen, nitro, cyano or an -OSO₂Y-group,
R⁴, R⁵ independently of one another are a -N₂⁺An⁻- or an -OSO₂Y-group,
R⁷ is hydrogen, methyl, ethyl or a phenyl residue substituted with the residues R⁹, R¹⁰, which is directly linked to the C atom bearing the R⁷,
R⁸ is hydrogen, ethyl, CN, NO₂, -CH₂CH₂-halogen or CH₂CH₂OH,
R⁹, R¹⁰ independently of one another are hydrogen, hydroxy, C₁-C₄-alkyl, C₁-C₄-alkoxy, halogen, nitro, cyano, a -N₂⁺An⁻- or an -OSO₂Y-group,
An⁻ is a halogen or ½ sulphate (SO₄²⁻) anion,
Y is OH or N(¹R)₂ and
R¹ is hydrogen, methyl or ethyl,
with the proviso that for each total molecule (3), one to two diazonium groups and one to two -OSO₂Y-groups are located on any arbitrary ring carbon atoms of aromatic rings,
and then the diazonium salts (3) obtained are reacted with dialkylamines of the formula
H N(R²)₂ (4)
wherein R² is methyl or ethyl,
in the presence of acid binding agents, optionally followed by preparation of the salts form the compounds obtained and optionally followed by liberating the hydroxyl and/or acid groups from these salts.

7. Diphenyl derivatives according to one of claims 1 to 5 for combating tumours of the skin and of sex hormone-dependent target organs in humans and animals.

8. Medicinal products containing at least one diphenyl derivative according to one of claims 1 to 5 and their salts, solvates and solvates of these salts, optionally together with one or more pharmacologically acceptable excipients or vehicles.

## Revendications

1. Dérivés de diphényle de formule 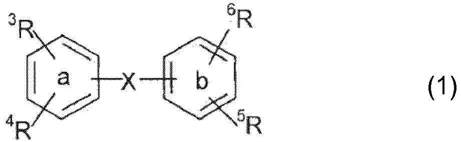 dans laquelle
X représente CO ou -CR⁷=CR⁸-,
R³, R⁶ représentent indépendamment l'un de l'autre un atome d'hydrogène, un groupe hydroxy, alkyle en C₁-C₄, alcoxy en C₁-C₄, alkyle en (C₁-C₄)-S-, alkyle en (C₁-C₄)-SO-, alkyle en (C₁-C₄)-SO₂-, un atome d'halogène, un groupe nitro, cyano ou -OSO₂Y,
R⁴, R⁵ représentent indépendamment l'un de l'autre un groupe -N=N-N(R²)₂ ou un groupe -OSO₂Y,
R⁷ représente un atome d'hydrogène, un groupe méthyle, éthyle ou un radical phényle substitué par les radicaux R⁹, R¹⁰, qui est lié directement à l'atome de carbone portant R⁷,
R⁸ représente un atome d'hydrogène, un groupe éthyle, CN, NO₂, -CH₂CH₂-halogène ou CH₂CH₂OH,
R⁹, R¹⁰ représentent indépendamment l'un de l'autre un atome d'hydrogène ou d'halogène, un groupe hydroxy, alkyle en C₁-C₄, alcoxy en C₁-C₄, nitro, cyano, un groupe -N=N-N(R²)₂ ou un groupe -OSO₂Y,
Y représente OH ou N(R¹)₂,
R¹ représente un atome d'hydrogène, un groupe méthyle ou éthyle et
R² représente un groupe méthyle ou éthyle,
à condition que par molécule (1) totale, un ou deux groupes -N=N-N(R²)₂ et un ou deux groupes -OSO₂Y se trouvent sur des atomes de carbone quelconques formant le cycle de noyaux aromatiques,
ainsi que leurs sels, acides libres, produits de solvatation et les produits de solvatation de ces sels et les produits de solvatation de ces acides libres.

2. Dérivés de diphényle selon la revendication 1 de formule 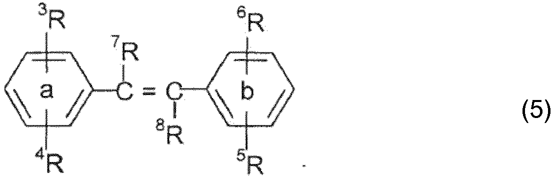 dans laquelle
R³, R⁶ représentent indépendamment l'un de l'autre un atome d'hydrogène, un groupe hydroxy, alkyle en C₁-C₄, alcoxy en C₁-C₄, alkyle en (C₁-C₄)-S-, alkyle en (C₁-C₄)-SO-, alkyle en (C₁-C₄)-SO₂-, un atome d'halogène, un groupe nitro, cyano ou -OSO₂Y,
R⁴, R⁵ représentent indépendamment l'un de l'autre un groupe -N=N-N(R²)₂ ou un groupe -OSO₂Y,
R⁷ représente un atome d'hydrogène, un groupe méthyle, éthyle ou un radical phényle substitué par les radicaux R⁹, R¹⁰, qui est lié directement à l'atome de carbone portant R⁷,
R⁸ représente un atome d'hydrogène, un groupe éthyle, CN, NO₂, -CH₂CH₂-halogène ou CH₂CH₂OH,
R⁹, R¹⁰ représentent indépendamment l'un de l'autre un atome d'hydrogène ou d'halogène, un groupe hydroxy, alkyle en C₁-C₄, alcoxy en C₁-C₄, nitro, cyano, un groupe -N=N-N(R²)₂ ou un groupe -OSO₂Y,
Y représente OH ou N(R¹)₂,
R¹ représente un atome d'hydrogène, un groupe méthyle ou éthyle et
R² représente un groupe méthyle ou éthyle,
à condition que par molécule (5) totale, un ou deux groupes -N=N-N(R²)₂ et un ou deux groupes -OSO₂Y se trouvent sur des atomes de carbone quelconques formant le cycle de noyaux aromatiques,
ainsi que leurs sels, acides libres, produits de solvatation et les produits de solvatation de ces sels et les produits de solvatation de ces acides libres.

3. Dérivés de diphényle selon la revendication 1, de formule 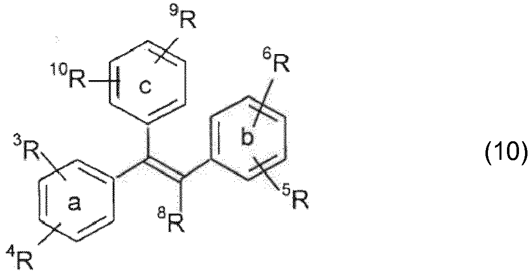 dans laquelle
R³, R⁶ représentent indépendamment l'un de l'autre un atome d'hydrogène, un groupe hydroxy, alkyle en C₁-C₄, alcoxy en C₁-C₄, alkyle en (C₁-C₄)-S-, alkyle en (C₁-C₄)-SO-, alkyle en (C₁-C₄)-SO₂-, un atome d'halogène, un groupe nitro, cyano ou -OSO₂Y,
R⁴, R⁵ représentent indépendamment l'un de l'autre un groupe -N=N-N(R²)₂ ou un groupe -OSO₂Y,
R⁸ représente un atome d'hydrogène, un groupe éthyle, CN, NO₂, -CH₂CH₂-halogène ou CH₂CH₂OH,
R⁹, R¹⁰ représentent indépendamment l'un de l'autre un atome d'hydrogène ou d'halogène, un groupe hydroxy, alkyle en C₁-C₄, alcoxy en C₁-C₄, nitro, cyano, un groupe -N=N-N(R²)₂ ou un groupe -OSO₂Y,
Y représente OH ou N(R¹)₂,
R¹ représente un atome d'hydrogène, un groupe méthyle ou éthyle et
R² représente un groupe méthyle ou éthyle,
à condition que par molécule (10) totale, un ou deux groupes -N=N-N(R²)₂ et un ou deux groupes -OSO₂Y se trouvent sur des atomes de carbone quelconques formant le cycle de noyaux aromatiques,
ainsi que leurs sels, acides libres, produits de solvatation et les produits de solvatation de ces sels et les produits de solvatation de ces acides libres.

4. Dérivés de diphényle selon la revendication 1 de formule 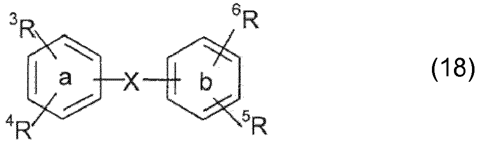 dans laquelle
X représente CO,
R³, R⁶ représentent indépendamment l'un de l'autre un atome d'hydrogène, un groupe hydroxy, alkyle en C₁-C₄, alcoxy en C₁-C₄, alkyle en (C₁-C₄)-S-, alkyle en (C₁-C₄)-SO-, alkyle en (C₁-C₄)-SO₂-, un atome d'halogène, un groupe nitro, cyano ou -OSO₂Y,
R⁴, R⁵ représentent indépendamment l'un de l'autre un groupe -N=N-N(R²)₂ ou un groupe -OSO₂Y,
Y représente OH ou N(R¹)₂,
R¹ représente un atome d'hydrogène, un groupe méthyle ou éthyle et
R² représente un groupe méthyle ou éthyle,
à condition que par molécule (18) totale, un ou deux groupes -N=N-N(R²)₂ et un ou deux groupes -OSO₂Y se trouvent sur des atomes de carbone quelconques formant le cycle de noyaux aromatiques,
ainsi que leurs sels, acides libres, produits de solvatation et les produits de solvatation de ces sels et les produits de solvatation de ces acides libres.

5. Dérivé de diphényle selon la revendication 1 ou 4, le dérivé de diphényle étant la 4-(3,3-diméthyl-triazényl-1)-4'-sodiosulfo-oxy-benzophénone selon la formule suivante

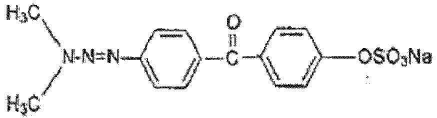

6. Procédé pour la préparation de dérivés de diphényle qui contiennent par molécule (i) au moins un groupe dialkyltriazényle et (ii) au moins un groupe sulfo-oxy et/ou au moins un groupe sulfamoyloxy, et de leurs sels, produits de solvatation et des produits de solvatation de ces sels, **caractérisé en ce qu'**on soumet à une diazotation des dérivés aminophényle de formule 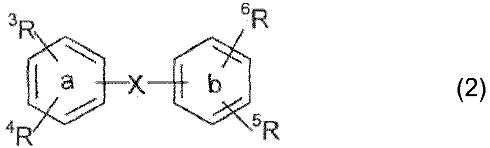 dans laquelle
X représente CO ou -CR⁷=CR⁸-,
R³, R⁶ représentent indépendamment l'un de l'autre un atome d'hydrogène, un groupe hydroxy, alkyle en C₁-C₄, alcoxy en C₁-C₄, alkyle en (C₁-C₄)-S-, alkyle en (C₁-C₄)-SO-, alkyle en (C₁-C₄)-SO₂-, un atome d'halogène, un groupe nitro, cyano ou -OSO₂Y,
R⁴, R⁵ représentent indépendamment l'un de l'autre un groupe -NH₂ ou un groupe -OSO₂Y,
R⁷ représente un atome d'hydrogène, un groupe méthyle, éthyle ou un radical phényle substitué par les radicaux R⁹, R¹⁰, qui est lié directement à l'atome de carbone portant R⁷,
R⁸ représente un atome d'hydrogène, un groupe éthyle, CN, NO₂, -CH₂CH₂-halogène ou CH₂CH₂OH,
R⁹, R¹⁰ représentent indépendamment l'un de l'autre un atome d'hydrogène ou d'halogène, un groupe hydroxy, alkyle en C₁-C₄, alcoxy en C₁-C₄, nitro, cyano, un groupe -NH₂ ou un groupe -OSO₂Y,
Y représente OH ou N(R¹)₂ et
R¹ représente un atome d'hydrogène, un groupe méthyle ou éthyle,
à condition que par molécule (2) totale, un ou deux groupes -NH₂ et un ou deux groupes -OSO₂Y se trouvent sur des atomes de carbone quelconques formant le cycle de noyaux aromatiques,
en présence d'acide fort, à basse température, en solution acide-aqueuse, avec un agent de diazotation, pour aboutir à des sels de diazonium de formule 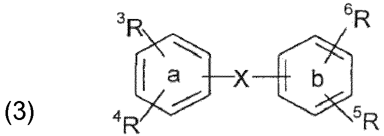 dans laquelle
X représente CO ou -CR⁷=CR⁸-,
R³, R⁶ représentent indépendamment l'un de l'autre un atome d'hydrogène, un groupe hydroxy, alkyle en C₁-C₄, alcoxy en C₁-C₄, alkyle en (C₁-C₄)-S-, alkyle en (C₁-C₄)-SO-, alkyle en (C₁-C₄)-SO₂-, un atome d'halogène, un groupe nitro, cyano ou -OSO₂Y,
R⁴, R⁵ représentent indépendamment l'un de l'autre un groupe -N₂⁺ An⁻ ou un groupe -OSO₂Y,
R⁷ représente un atome d'hydrogène, un groupe méthyle, éthyle ou un radical phényle substitué par les radicaux R⁹, R¹⁰, qui est lié directement à l'atome de carbone portant R⁷,
R⁸ représente un atome d'hydrogène, un groupe éthyle, CN, NO₂, -CH₂CH₂-halogène ou CH₂CH₂OH,
R⁹, R¹⁰ représentent indépendamment l'un de l'autre un atome d'hydrogène ou d'halogène, un groupe hydroxy, alkyle en C₁-C₄, alcoxy en C₁-C₄, nitro, cyano, un groupe -N₂⁺ An⁻ ou un groupe -OSO₂Y,
An⁻ représente un anion halogénure ou un ½ anion sulfate (SO₄²⁻₎,
Y représente OH ou N(R¹)₂ et
R¹ représente un atome d'hydrogène, un groupe méthyle ou éthyle,
à condition que par molécule (3) totale, un ou deux groupes diazonium et un ou deux groupes -OSO₂Y se trouvent sur des atomes de carbone quelconques formant le cycle de noyaux aromatiques,
et ensuite on fait réagir les sels de diazonium (3) obtenus avec des dialkylamines de formule
H N(R²)₂ (4)
dans laquelle R² représente le groupe méthyle ou éthyle,
en présence de capteur d'acide, ce qui est éventuellement suivi de la préparation des sels à partir des composés obtenus et éventuellement suivi de la libération des groupes hydroxy et/ou acides à partir de ces sels.

7. Dérivés de phényle selon l'une quelconque des revendications 1 à 5, destinés à combattre des tumeurs de la peau et des organes cibles dépendant d'hormones sexuelles chez les êtres humains et les animaux.

8. Médicament, contenant au moins un dérivé de diphényle selon l'une quelconque des revendications 1 à 5 et ses sels, produits de solvatation et produits de solvatation de ces sels, éventuellement conjointement avec un ou plusieurs excipients ou véhicules pharmacologiquement acceptables.
